# EUROPEAN PATENT SPECIFICATION

(11) **EP 0 406 357 B1**
(45) Date of publication and mention of the grant of the patent: **07.01.1998**
(21) Application number: 89913054.6
(22) Date of filing: 07.11.1989
(51) Int. Cl.: C12N 1/20, C12N 15/09, C12P 21/02, C07H 15/12, C07K 14/00

(54) **FUNCTIONAL RECOMBINANTLY PREPARED SYNTHETIC PROTEIN POLYMER**
FUNKTIONELLES, DURCH EIN REKOMBINANTES VERFAHREN HERGESTELLTES SYNTHETISCHES PROTEINPOLYMER
POLYMERE FONCTIONNEL A BASE DE PROTEINE SYNTHETIQUE PRODUIT GRACE A UN PROCEDE RECOMBINANT

(30) Priority: 09.11.1988 US 269429
(43) Date of publication of application: 09.01.1991
(73) Proprietor: PROTEIN POLYMER TECHNOLOGIES, INC., San Diego, CA 92121 (US)
(72) Inventor: CAPPELLO, Joseph, San Diego, CA 92122 (US); FERRARI, Franco, A., LaJolla, CA 92037 (US)
(74) Representative: Harrison, David Christopher
(86) International application number: US8905016
(87) International publication number: WO9005177

(56) References cited:
- WO-A-88/03533
- GB-A- 2 162 190
- US-A- 4 187 852
- US-A- 4 474 851
- US-A- 4 589 882
- Chemical Abstracts, Volume 93, Number 9, issued 01 September 1980. B. Nakajima, et al. see page 685, abstract number 95634s. Peptide Chem. 1979 (Pub 1980) 17th, 169-74. see entire abstract.
- Cell, Volume 44, published 28 February 1986. E. Ruoslahti, et al. pp 517-518, see entire article.
- Gene, Volume 39, published September 1985. T. Kempe, et al. pp 239-245. see entire document

## Description

This invention is related to the production of high-molecular-weight polymers of amino acids based on biologically and chemically active structural polymers.

Recombinant DNA technology has been applied in the isolation of natural genes and the expression of these genes in a variety of host cells. Typically, this technology has had utility in producing biologically active polypeptides, such as interferons or peptide hormones, which were impractical to produce in useful amounts by other means. It was also possible to produce modified proteins by isolating natural genes and utilizing the techniques of site specific, in vitro mutagenesis to alter these genes and thereby change the polypeptides produced. Other polypeptides have been created by combining sections of various native genes to produce new polypeptides that are chimeric molecules of the several naturally occurring molecules.

With the advent of efficient and automated methods for the chemical synthesis of DNA, it has become possible to synthesize entire genes and to modify such synthetic genes at will during the course of synthesis. However, these various technologies have been applied to the production of natural or modified versions of natural polypeptides. There have been very few attempts to use these technologies to create substantially new polypeptides. In nature, polypeptides have a wide range of chemical, physical and physiological characteristics. Nevertheless there are commercial applications for which known, naturally occurring polypeptides are not appropriate.

While biotechnology is versatile, usually it has been limited in its applications to naturally occurring products or modifications of naturally occurring molecules. One great strength of organic chemical synthesis, by contrast, has been the ability to transform inexpensive carbon materials to a wide variety of polymeric molecules, including naturally occurring molecules, but most importantly entirely new chemical structures, such as polypropylene and polyacrylates, which have defined and predicted chemical properties not associated with naturally occurring molecules.

Such materials, particularly high-molecular-weight polymers containing repeating sequences of amino acids, have proven difficult to produce by biochemical means. The genes necessary for producing large peptides containing repeating units of amino acids were unstable and often underwent intermolecular recombination causing deletions of repeating units in the gene. The development of a biotechnology which would produce polymeric molecules by biological processes similar to those available by organic synthesis would significantly broaden the range of applications of biotechnology.

### Brief Description of the Relevant Literature

The cloning of multiple lactose operators up to four in tandem is disclosed by Sadler et al., Gene, (1980) 8:279-300, Hybrid bacterial plasmids containing highly repeated satellite DNA is disclosed by Brutlag et al., Cell, (1977) 10:509-519. The synthesis of a poly(aspartyl-phenylalanine) in bacteria is disclosed by Doel et al. Nucleic Acids Research, (1980) 8:4575-4592. A method for enriching for proline content by cloning a plasmid which codes for the production of a proline polymer was disclosed by Kangas et al., Applied and Environmental Microbiology, (1982) 43:629-635. The biological limitations on the length of highly repetitive DNA sequences that may be stably maintained within plasmid replicons is discussed by Gupta et al. in Bio/Technology, p. 602-609, September 1983. WO 88/03533 discloses a high molecular weight polymer containing repeating amino acid sequence units or strands thereof.

In contrast, in the present invention, the structural components of repetitive oligomeric units or strands are separated by different amino-acid sequences having particular functional capabilities. (By "strands" is intended an ordered sequence capable of alignment with a second strand having substantially the same or a complementary sequence e.g., hydrophobic aligns with hydrophobic and hydrophilic aligns with hydrophilic.) The components of the repetitive units provide a structure which allows for availability of the intervening units for interacting with the environment, such as solutions, gases, gels and the like where the intervening sequence is substantially free of steric inhibition to interact with other molecules as compared to the strands. Long nucleic-acid sequences are built up by synthesizing nucleic-acid oligomers which express a plurality of individual repetitive peptide units and the oligomers are joined to provide a polynucleotide of the desired length. By providing for specific restriction sites which are relatively evenly spaced, additional sequences may be introduced at sites which provide for turns or other functional entity between repetitive strands. The resulting nucleic-acid open-reading frames may then be introduced into an appropriate expression vector for expression of the desired protein product.

Novel polypeptides are provided which are polyoligomers of repeating, relatively short, amino acid sequence units forming strands, where the strands are separated by a different oligomeric unit which results in a sequence available to the environment of the polypeptide for a variety of functions: the intervening oligomers are unaligned and comprise a binding or chemically active amino acid sequence.

The polypeptides which are encoded for by the nucleic-acid sequences of the subject invention provide for strands which may align to provide for a basic structure. The strands will be protein chain segments of more or less linear conformation. The strands may be aligned in reverse or direct alignment, where a plurality of strands are separated by a sequence other than the repetitive sequence as a functional entity between the strands, so as to be accessible to solutes or components in the medium. The strands will be based primarily on repeating units of naturally occurring polymers, where the repeating units generally are at least three amino acids and may include the same amino acid twice.

The polymers of this invention may be used to provide a variety of structures for a variety of purposes. Depending on the repeating units and their relationship to known polymeric structures, analogous mechanical, e.g., tensile properties may be achieved, which may be modified for a particular intended purpose. The subject polymers may be used by themselves to produce fibers, films, membranes, adhesives, emulsions, and the like or with other compounds or compositions to form composites, laminates or combinations of the aforementioned products.

The structurally aligned elements of the polymers may be β-sheets, α-helices, dynamic β-spiral, collagen helix, Type I or II, or combinations thereof, where there will be intervening elements which will be of different sequence and structure and will usually not participate in the aligned elements. For the most part, these intervening sequences will be loops or turns.

The genes of the subject invention comprise multimers of DNA sequences encoding the same amino acid sequence unit, where two or more different multimers encoding different amino acid units may be joined together to form a block copolymer. The individual units may have from 3 to 30 amino acids (9 to 90 nt), more usually 3 or 4 to 25 amino acids (9 to 75 nt), particularly 3 or 4 to 15 amino acids (9 to 45 nt), more particularly 3 or 4 to 8 amino acids (9 to 24 nt), usually having the same amino acid appearing at least twice in the same unit, generally separated by at least one amino acid. The units of the multimer coding for the same amino acid sequence may involve two or more nucleotide sequences, relying on the codon redundancy to achieve the same amino acid sequence.

The units of the strands will generally be at least about 25 amino acids and not more than about 150 amino acids, usually not more than about 100 amino acids, preferably from about 30 to 75 amino acids. The other sequence separating the strands will generally be at least about 4, more usually 6 amino acids and not more than about 50, usually not more than about 30 amino acids, more usually not more than about 20 amino acids, where tne longer sequences are associated with parallel strands rather than antiparallel strands, with alignment of the strands in the N to C direction.

Various natural proteins have repeating structures, such as collagen with a G-X-Y (wherein X equals any amino acid, often ala or pro, and Y equals any amino acid, often pro or hydroxy-pro) repeating unit, elastin with VPGG, VPGVG, and/or PGVGV units, keratin with a so called "heptad" repeat unit consisting of a seven amino acid oligopeptide with positions one and four occupied consistently with hydrophobic aliphatic or aromatic residues, e.g., AKLKLAE or AKLELAE, and mussel adhesive with a decapeptide or hexadecapeptide coming within the sequence A-K-P-P∗-S-T-Y-X-P-P*-P-S-T-Y-X-K (P* is hydroxyproline and X is an aromatic amino acid, particularly L-dopa; see US-A- 4,585,585 and 4,687,740). The repeating units may be used individually or in combination, where individual units may alternate in a particular pattern or individual strands may be provided.

Of particular interest are polypeptides which have as a repeating unit SGAGAG (G = glycine; A = alanine; S = serine). This repeating unit is found in a naturally occuring silk fibroin protein, which can be represented as GAGAG(SGAGAG)₈SGAAGY (Y = tyrosine).

For the intervening oligomers or turns between the strands, various sequences may be used, depending upon the desired purpose of the polymer. Thus, the intervening sequence may be unaligned, flexible, accessible, functional or combinations thereof. Thus, the intervening sequence in association with the strand sequence can be designed to provide a wide variety of products which may be formed, fabricated, extruded, spun, woven, coated, or the like. The intervening sequence may provide for a ligand, which may serve to bind to antibodies, naturally occurring receptors, non-amino-acid molecules, or the like. In this way, the polymeric structures may be used to specifically bind a wide variety of molecules serving as affinity columns, use in diagnosis, sensors, cell separation, device coatings having, for example, antithrombogenic properties, cell substrates, and the like.

The intervening sequence may provide chemically active amino acids for chemical crosslink sites, which may serve to covalently attach functional peptides, synthetic or natural polymers or proteins, non-amino acid molecules, and the like. The intervening sequence may be a naturally occurring sequence or a modified naturally occurring sequence. Naturally occurring sequences may be derived from a wide variety of sources with a variety of functions. Such sequences may be a cellular growth inhibitor sequence, e.g., from tenascin (Chiquet-Ehrismann et al., (1988) Cell 53:383-390; cell growth promoting attachment factors, e.g., from fibronectin, -RGD-, -REDV-); Humphries et al., (1988) J. Cell Biol. 103:2637-2647) fibronectin (-RGD-); Suzuki et al., (1985) EMBO J. 4:2519-2524, laminin B1 (-YIGSR); WO 89/03392, collagen; Graf et al., (1987) Cell 48:989-996, vitronectin (-XAP-); bacterial adhesive (-SLF-, -AIF-) (Jacobs et al., (1987) J. Bacteriology 1691:735-741; growth hormone, and insulin; cellular function activators, such as major histocompatibility complex antigens, Class I and II, particularly the α₁, α₂, β₁, and β₂ regions, e.g., HLA-A2 amino acids 50-80 and 140-170 (Bjorkman et al., (1987) Nature 329:512-518) and HLA-D amino acids 1-90 (Todd et al., (1988) Science 240:1003-1009); growth factor domains, e.g., EGF, TGF and VGF, IL-1-8, particularly -2 and -4, and erythropoietin; viral attachment sequences, such as human CD4 amino acids 35-60 (Clayton et al., (1988) Nature 335:363-366) and 70-95 (Lifson et al., (1988) Science 241:712-716); sequences which promote the binding of non-protein molecules, such as the heparin binding domain of vitronectin, metal binding domains, e.g., metallothioneins, glucose and other sugar binding domains, e.g., lectins, B chains of toxins, such as abrin, ricin, and diphtheria toxin, safratoxin, or fragments thereof, etc., and drug or toxin binding domains for detoxification; and chemically active amino acids or amino acid sequences for post-translational modifications, such as N-X-S for N-linked glycosylation and the amino acids, C, M, H, K, R, D, E, W, F, Y, N and Q for chemical modification.

Sequences of specific interest as intervening sequences include: 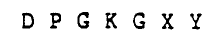 wherein at least one of X and Y is C; 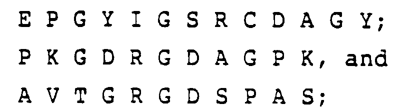 where conservative substitutions may be made at other than the functional site.

For the cysteine product it will be desirable to have two or three cysteines in a multimer unit, preferably having a cysteine proximal to each end of the multimer unit. For chemical cleavage the dipeptide DP or EP is desirable.

Other sequences of interest may be epitopes of microorganisms, e.g., virus, bacteria, fungi and protozoa, phosphorylation sites, peptidase recognition sites or the like.

The use of genetic engineering in the preparation of copolymers involving repeating units with intervening sequences is a powerful method for varying properties, by appropriate choice of the different units, the number of units in each multimer, the spacing between them, and the number of repeats of the multimer combination assembly. Thus, by varying the number and arrangement of the primary multimers, a variety of different physical and chemical properties can be achieved.

Exemplary of the use of the block copolymers are combinations of silk units and elastin units to provide products having properties distinctive from polymers only having the same monomeric unit.

To prepare the structural genes, various approaches can be employed. To prepare the oligomers, complementary strands of DNA may be synthesized, so that upon hybridization double-stranded DNA is obtained with the appropriate termini. If desired, each of the oligomeric units may be the same, so that in a single step, a concatemer may be obtained depending upon the conditions of the hybridization. Normally, conventional annealing and ligating conditions will be employed, such as are described in the examples that follow.

If desired, two different oligomeric units may be prepared where the termini of the two units are complementary one with the other, but the termini of the same unit are unable to bind together. In this way one can build individual oligomeric units and then join them together to form the concatemer, where the intervening linking sequences are defined at least in part by the termini. Depending upon the construct, the 5' terminus may provide for the initiation codon methionine, or the structural gene may be joined to an adapter which may provide for a unique sequence (optionally cleavable by enzymatic or chemical treatment) at the 5' terminus or may be inserted into a portion of a gene, usually endogenous to the host, in proper reading frame so as to provide for a fusion product. By providing for appropriate complementary termini between the adapter or truncated gene and the 5' end of the subject structural gene, the sequences can be joined in proper reading frame to provide for the desired protein. Advantages that may be achieved by employing adapters or fusion proteins include having specific sequences for special purposes, such as linking, secretion, complex formation with other proteins, affinity purification, or the like. The 3' region may be similarly modified to provide analogous functions.

Once the structural gene has been assembled, it may be cloned; clones having the desired gene, particularly as to sequence length, may be isolated; and the gene may be removed and used to join to a sequence for expression.

The expression construct will include transcriptional and translational initiation and termination regulatory regions, 5' and 3', respectively, of the structural gene. As already indicated, these regions may be created by employing a fusion protein, where the subject structural gene is inserted into a different structural gene downstream from its initiation codon and in reading frame with the initiation codon. Alternatively, various transcriptional and translational initiation regions are available from a wide variety of genes for use in expression hosts, so that these transcriptional and translational initiation regions may be joined to the subject structural gene to provide for transcription and translation initiation of the subject structural genes. A wide variety of termination regions are available which may be from the same gene as the transcriptional initiation region or from a different gene. Numerous constructs have been disclosed in the literature, and the same procedures may be applied with the subject gene as have been employed with other structural genes.

Of particular interest is the use of an inducible transcription initiation region. In this manner, the host strain may be grown to high density prior to significant expression of the desired product. Providing for inducible transcription is particularly useful where the peptide is retained in the cellular host rather than secreted by the host.

A number of inducible transciption initiation regions exist or can be employed in particular situations. The inducible regions may be controlled by a particular chemical, such as isopropyl thiogalactoside (IPTG) for inducing the beta-galactosidase gene. Other inducible regions include lambda left and right promoters; various amino acid polycistrons, e.g., histidine and tryptophan; temperature sensitive promoters; and regulatory genes, e.g., cI^{ts} 857.

An alternative system which may be employed with advantage is use of a combination of transcription initiation regions. A first transcription initiation region which regulates the expression of the desired gene but which is not functional in the expression host by failing to be functional with the endogenous RNA polymerase is employed. A second transcription initiation region, such as an inducible region, can then be employed to regulate the expression of an RNA polymerase with which the first transcription initiation region is functional. In this manner expression only occurs upon activation of the regulatory region controlling the expression of the exogenous RNA polymerase. This system may be illustrated with the T7 phage transcription initiation region, specifically the initiation regions of genes 9 and 10 of T7 phage.

An alternative system relies on the use of mutants which undergo a developmental change based on a change in the environment, such as a lack of a nutrient, temperature, osmotic pressure, salinity, or the like. Illustrative of this system, strains of B. subtilis can be obtained which are incapable of sporulation but which can produce those components which initiate expression of products involved with sporulation. Therefore, by a change in the condition of the medium, a transcription initiation region associated with sporulation will be activated. In this situation, the host provides the necessary inducing agent or activator to initiate expression.

Various other techniques exist for providing for inducible regulation of transcription and translation of a gene in a particular host.

For the most part, the host will be a unicellular organism, either a prokaryote or a eukaryote, selected from bacteria, algae, fungi, filamentous fungi, plant or animal tissue culture cells, etc. Illustrative hosts include E. coli, B. subtilis, B. stearothermophilus, S. cerevisiae, Aspergillus, Neurospora, CHO cells, HeLa cells, etc. and the like. Alternatively, whole plants may be employed.

The expression construct for expression of the desired gene, by itself or in conjunction with any auxiliary genes involved with transcription, will normally be joined to an appropriate vector for introduction into the expression host. A large number of vectors are commercially available with others being described in the literature. The vectors are normally characterized by having one or more unique restriction sites, a replication system for extrachromosomal maintenance in the host, and one or more markers which allow for selective pressure on the host. The markers may provide complementation, prototrophy to an auxotrophic host, resistance to a biocide, e.g., an antibiotic such as penicillin or kanamycin, or the like. In some instances, rather than selective pressure, a marker gene is employed which allows for detection of particular colonies containing the gene. This situation is illustrated by the gene for beta-galactosidase, where a substrate is employed which provides for a colored product.

The expression construct, including any auxiliary genes, may be introduced into the expression vector in accordance with known techniques, particularly employing restriction, insertion, and ligation.

The expression construct may then be used for transformation of the appropriate host. Depending upon the host, either intact cells or protoplast may be employed, where transformation or conjugation is employed. Conveniently, calcium-phosphate-precipitated DNA or non-ionic detergents may be employed to introduce the plasmid into the host. It should be appreciated that it is not necessary to employ vectors for host transformation, since bare DNA can be introduced for integration into the genome. However, even where integration is desired, a much greater efficiency of integration is achieved employing the vectors, thus favoring the employment of vectors.

Depending upon the nature of the vector, the expression construct may be maintained on an extrachromosomal element or become integrated into the host. Where integration is desired, it will usually be desirable with prokaryotes and some eukaryotes to have a sequence homologous to a sequence in the chromosome of the host. Usually the sequence will be at least about 200 bp and not more than about 5000 bp, usually not more than about 2000 bp. The choice of the homologous sequence is somewhat arbitrary, but may be used for complementation, where the host is an auxotrophic mutant and the homology provides prototrophy.

The transformants or integrants may then be grown in an appropriate nutrient medium to high density, followed by induction of transcription in accordance with the nature of the transcriptional system of the expression construct. Where the desired protein is retained in the cytoplasm, these cells are harvested and lysed, and, depending upon the use of the protein, the protein may be further purified in accordance with conventional techniques, such as chromatography, solvent-solvent extraction, affinity chromatography, and the like.

The following examples are offered by way of illustration and not by way of limitation.

### EXPERIMENTAL

### Example 1

### DNA Preparation Methods

1. Preparation of plasmid DNA from E. coli.
   A. Small scale. Plasmid DNA was prepared from 1.5 ml cultures by either the boiling procedure or the alkaline lysis method (Maniatis et al., Molecular Cloning: A Laboratory Manual. Cold Spring Harbor Laboratory, Cold Spring Harbor. (1982)).
   B. Large scale. A plasmid-carrying strain was grown overnight in 1 liter of Luria broth with the appropriate antibiotic. The cells were collected by centrifugation at 10,000xg for 5 min and resuspended in 10 ml of ice cold TE (10 mM TRIS-HCl pH 8, 1 mM EDTA). The cells were centrifuged again, resuspended in 4 ml of TES (TE and 25% w/v sucrose) and homogenized by vortexing. The samples were kept on ice for the following steps. Lysozyme (1 ml of 10 mg/ml) was added to the cell suspension and incubated for 5 min before the addition of 2 ml of 0.5 M EDTA pH 8. After 10 min incubation, 50 ml of proteinase K (40 mg/ml) were added followed 10 min later with 15 ml of lysing buffer (0.1% Triton X-100, 1 mM EDTA, 50 mM TRIS-HCl pH 8). After 15-20 min, the cell lysate was centrifuged at 35,000xg for 90-120 min. The supernatant (19.8 ml) was transferred to a plastic tube with 20 g of CsCl and 400 µl of ethidium bromide (10 mg/ml). After dissolution, the mixture was divided into two polyallomer ultracentrifuge tubes, sealed with heat and centrifuged in a Beckman Ti 65 motor at 60,000 rpm for 24 hr. The lower plasmid DNA band was removed from the tube with a hypodermic needle. The ethidium bromide was extracted three times with an equal volume of NaCl-saturated isopropanol. Two volumes of H₂O were added to the DNA solution, and then the DNA was precipitated with ethanol.
2. Preparation of double-stranded DNA. A culture of JM103 was grown to an OD₆₀₀ of about 0.2 and then divided into aliquots of 2 ml. Each aliquot was infected with a fresh plaque of M13 and incubated at 37°C for about 6 hr with vigorous shaking. Then the cells were pelleted and the supernatant was saved for subsequent infections. The double-stranded phage DNA was extracted by the boiling method (Maniatis et al.).
3. Deproteinization. Phenol extraction was performed on a convenient volume of DNA sample, typically between 100 µl to 10 ml. The DNA sample was diluted in 0.01 M TRIS-HCl pH 7.5, 1 mM EDTA and an equal volume of water-saturated phenol was added. The sample was vortexed briefly and placed on ice for 3 min. After centrifugation for 3 min in a microfuge, the aqueous layer was removed to a new tube and extracted once with an equal volume of chloroform:isoamylalcohol (24:1).
4. Ethanol precipitation. DNA in an aqueous buffer was concentrated by ethanol precipitation. To the DNA sample was added 1/10 volume of 3 M sodium acetate pH 7.5 and 2-3 volumes of cold ethanol. The DNA was precipitated for 30 min at -70°C or overnight at -20°C and then pelleted by centrifugation in the microfuge for 15 min at 4°C. The pellet was washed once with 200 µl of cold 80% ethanol and pelleted again for 10 min at 4°C. After air drying or lyophilization, the pellets were resuspended in the appropriate buffer.
5. Phosphatase treatment of DNA. Phosphatase treatment of DNA was performed by adding 1 µl (25 units) of calf intestinal phosphatase (Boehringer Mannheim) directly to the restriction enzyme digestion reaction and continuing the incubation for 30 min at 37°C. The phosphatase was inactivated for 60 min at 65°C prior to deproteinization by phenol extraction.
6. Fill-in reaction with DNA polymerase I. DNA was resuspended in buffer containing 50 mM TRIS-HCl pH 7.4, 50 mM KCl, 5 mM MgCl₂, and 400 µM each of the four deoxynucleotide triphosphates. Ten units of Klenow DNA polymerase (BRL) were added, and the reaction was allowed to proceed for 15 min at room temperature. The DNA was then phenol extracted and ethanol precipitated.
7. T4 polynucleotide kinase reaction. The reaction (10 µl) contained: T4 polynucleotide kinase (BRL), 150 ng of DNA, 1 µl of 10x kinase buffer (0.7 M TRIS-HC1 pH 7.6, 0.1 M MgCl₂, 50 mM DTT) and [³²P]-ATP (200-300 µCi). This was incubated at 37°C for 30 min and then the DNA was purified using a NACS column (Bethesda Research Labs).
8. Digestion with restriction endonucleases. DNA was digested with restriction endonucleases (REN) in 1 x "AA" buffer [10 x AA buffer is 330 mM TRIS-acetate, pH 7.9, 660 mM potassium acetate, 100 mM magnesium acetate, 50 M dithiothreitol (DTT) and 1 mg/ml bovine serum albumin (nuclease free)]. Whenever possible, the concentration of DNA was kept below 1 µg/25 µl. Incubation was at 37°C for 1-4 hrs for most restriction endonucleases except for BalI, BanI and NaeI digestions which were incubated overnight.
9. Analytical agarose gel electrophoresis of DNA. To DNA samples for gel analysis we added 0.2 volumes of loading buffer (5 x electrophoresis buffer, 0.01% bromphenol blue dye, 50 mM EDTA, and 50% glycerol). Then the samples were loaded into lanes of a horizontal submerged electrophoresis unit containing a 1.0% (w/v) agarose gel. The electrophoresis buffer was either 1 x TAC or 1/2 x TBE. The 1 x TAC is 40 mM TRIS-base, 10 mM EDTA, adjusted to pH 7.8 with acetic acid. The 1/2 x TBE is 0.045 M TRIS-base, 0.045 M bor c acid, 1 mM EDTA, pH 8. The gel was run at 40-50 V for 18 hr, then removed and stained with 0.5 µg/ml ethidium bromide for 30 min. The DNA bands were visualized on a long wavelength UV transilluminator.
10. Preparative agarose gel electrophoresis. The procedures and materials are the same as for the analytical agarose gel electrophoresis. The only difference is the use of low melting point agarose, ranging in concentration from 0.5 to 2.5% (w/v) depending on the size of the DNA fragment to be purified. DNA restriction fragments were excised from the LMP agarose gels after visualization with ethidium bromide.
11. NACS purification. Gel fragments containing DNA were melted at 70°C for 5 min and diluted approximately 5 fold with TE1 (10 mM TRIS-HCl pH 7.5, 0.2 M NaCl). The gel solution was applied to a NACS column (BRL). The column was washed with 5 ml of the same buffer. The bound DNA was eluted with 300 µl of either TE2 (10 mM TRIS-HCl pH 7.5, 1.0 M NaCl) for DNA fragments smaller than 1000 bp or TE3 (10 mM TRIS-HCl pH 7.5, 2M NaCl) for larger fragments. The eluted DNA was concentrated by ethanol precipitation.
12. DNA ligation. Reactions for ligating cohesive ends contained: 1 µg DNA, 1 x AA buffer (see step 8, above) 1 mM ATP and 20 units of T4 DNA ligase (BRL) in a 20 µl final reaction volume. The ligation was allowed to proceed for 16-18 hr at 15°C or 1-2 hr at room temperature. For blunt-ended ligations the reactions contained 1 µg DNA, 25 mM TRIS-HCl pH 7.5, 5 mM MgCl₂, 5 mM DTT, 0.25 mM spermidine, 200 mg BSA, 1 mM hexamine cobalt chloride (HCC), 0.5 M ATP and 400 units T4 DNA ligase (NEB) in a 20 µl reaction volume. The ligation was allowed to proceed for 30 min to 1 hr at room temperature.

### Bacterial Transformation Methods

1. Preparation of transformation-competent E. coli cells. A culture of 200 ml of sterile L broth was inoculated with a small loopful of E. coli cells. This was incubated with shaking at 37°C until the OD₆₀₀ was approximately 0.5. The culture was placed on ice for 10 min and centrifuged at 6,000xg for 10 min. The cell pellet was resuspended in 100 ml of ice-cold 0.1 M MgCl₂, kept on ice for 30-40 min and centrifuged again. The pellet was resuspended in 2 ml of ice-cold 100 mM CaCl₂, transferred to a sterile test tube and incubated on ice for 24 hr. The competent cells were then aliquoted and stored at -70°C.
2. Transformation of E. coli. An aliquot of frozen competent cells were thawed on ice. To 50 µl of cells 0.1 to 1 µg of DNA was added and the mixture was incubated on ice for 30 min. The tube was removed from ice and placed in a 42°C bath for 2 min. L broth (1 ml) was added and the transformation mix incubated with shaking at the desired temperature (usually 30°C or 37°C) for 2 hr. Then one-tenth of the transformation was plated on L broth plates containing the appropriate antibiotic and, when necessary, XGAL and IPTG were added.

### Antibody Production, Protein Chemistry and Electrophoresis of Proteins

1. Preparation of antibody to artificially synthesized peptides. Synthetic peptide of sequence (GAGAGS)₈GAAGY was coupled to BSA using the glutaraldehyde procedure of Kagen and Glick (1979). The degree of coupling was monitored using trace amounts of radioactive iodinated synthetic peptide. Peptide conjugates at a concentration of 1 mg/ml in complete Freund's adjuvent were used to immunize rabbits at day 0. Animals were re-injected with antigen in Freund's incomplete, adjuvant at day 30 and titered at day 60. Positive sera was detected using a microtiter RIA using the synthetic peptide as antigen. Kagen and Glick (1979), in Methods of Radioimmunoassay, Jaffe and Berman (eds.), Academic Press, p 328.
   A peptide of 53 amino acids corresponding to the SLPIII sequence (V-P-G-V-G)₈ was prepared on an Applied Biosystems peptide synthesizer. The yield of this material, which has a molecular weight of 3640 was approximately 0.5 grams. The peptide was coupled to bovine serum albumin. The material was sent to Antibodies, Inc. for preparation of antibodies in rabbits. Antisera was obtained that reacted with synthetic peptides of the SLPIII sequence. These antisera have been useful for the detection of fusion peptides containing gly-ala sequences.
   Following the procedure described above an additional antigen was synthesized having the formula YTITVYAVTGRGDSPASSKPISINYC of fibronectin (the FCB portion) and the sequence (GAP[GPP]₄)₂ (the CLP portion), which was coupled to keyhole limpet hemocyanin for use as an immunogen. Polyclonal antisera was then prepared as described above which bound to the FCB and CLP peptide.
2. Polyacrylamide gel electrophoresis of proteins. Approximately 10⁹ E. coli cells from growing cultures were pelleted by centrifugation at 10,000xg for 5 min. The cell pellets were resuspended in 100 to 500 µl of 2X sample buffer (100 mM TRIS-HCl pH 6.8, 4% SDS, 10% B-mercaptoethanol, 60% glycerol or sucrose) and sonicated for 30 sec using a Tekmar sonic disruptor. Samples were boiled for approximately 5 min and 20 to 100 µl of the cell lysates were loaded on an SDS-polyacrylamide gel (7.5 to 16% w/v). The gels were prepared following the procedure of Laemmli (Nature, _ 27:_80-685 (1970)). The proteins in the gels were stained with 2% Coomassie brilliant blue in 10% methanol, 7.5% acetic acid for 1 hr and destained in 10% methanol, 7.5% acetic acid overnight.
3. Immunoblotting of proteins in gels. After protein electrophoresis, one of the flanking glass plates was removed from the polyacrylamide gel. The gel surface was wetted with transfer buffer (25 mM TRIS-HCl, 192 mM glycine, 20% methanol). A piece of nitrocellulose paper (Sartorius, SM11307) was saturated with transfer buffer and laid on the gel. Air bubbles between the filter and the gel were removed. The gel and nitrocellullose filter were placed in the transfer unit as specified by manufacturer (Bio-Rad). Transfer was allowed to proceed at 200 mA for 3-4 hr. Then the nitrocellulose filter was removed and stained with Amido-Schwartz for 3 min (0.05% Amido black, 45% deionized H₂O), 45% methanol, 10% acetic acid) and destained in H₂O. The filter was incubated for at least 10 min at room temperature in "BLOTTO" (5% w/v nonfat dry milk, 50 mM TRIS-HCl pH 7.4, 0.9% w/v NaCl, 0.2% w/v sodium azide). The filter was placed in serum appropriately diluted (1:50 to 1:500) in 0.5X Blotto (2.5% nonfat dry milk, 50 mM TRIS-HCl pH 7.4, 0.9% NaCl, 0.2% sodium azide) and was gently agitated for approximately 16 hr at room temperature. The filter was washed for 1 hr with 5 changes of TSA (50 mM TRIS-HCl pH 7.4, 0.9% NaCl, 0.2% sodium azide). The blot was placed in 15 ml of 0.5X BLOTTO solution containing 1x10⁷ cpm of the ¹²⁵I -protein A and gently agitated for 2 hr at room temperature. The filter was washed for 2 hr with a minimum of 7 changes of TSA, rinsed once with deionized H₂O and air dried. The blot was covered with Saran wrap and autoradiographed.
4. Amino Acid Analysis. Amino acid compositions are determined by the PTC derivitization procedure of Henrickson and Meredith (1984). Protein samples were hydrolysed with 5.7 N constant boiling HCl at 108°C for 24 hours in vacuo. After reaction with PITC, amino acid derivatives were detected at 254 nm by HPLC reverse phase chromatography using a Hewlett Packard 1090 system and a Supelco C18 column (4.6 mm x 25 cm) with a linear gradient of 0-50% acetonitrile in 0.1 M NH₄OAc pH 6.78 as a mobile base. Henrickson, R.L. and Meredith, S.C. (1984) Amino Analysis by Reverse Phase High Performance Liquid Chromatography., Anal. Biochem. 137:65-74.
5. Amino Acid Sequence Analysis. The N-terminal amino acid sequence was determined by automated Edman degradation using an Applied Biosystems Model 470A gas phase protein sequenator. The PTH amino acid derivatives were analyzed by reverse phase HPLC using a Hewlett Packard 1090 system and an Altex C18 column (2 mm x 25 cm) with a complex gradient buffer system.
6. Peptide Synthesis. Synthetic peptides were prepared by solid phase synthesis on an Applied Biosystems Model 430A Peptide Synthesizer using the standard symmetric anhydride chemistry as provided by the manufacturer. The coupling yield at each step was determined by the quantitative ninhydrin procedure of Sarin et al., (1981). The synthetic peptide was cleaved from the solid support and amino acid blocking groups were removed using anhydrous HF (Stewart and Young, 1984). Crude peptides were desalted by chromatography over Sephadex G-50. Sarin, V.K., Kent, S.B.H., Tam, J.P. and Merrifield, R.B. (1981). Anal. Biochem. 237:927-936. Stewart, J.M. and Young, J.D. (1984). Solid Phase Peptide Synthesis, Pierce Chemical Company, Rockford, IL. pp 85-89.

### Synthetic DNA Methods

1. In vitro DNA synthesis. The N,N-diisopropylphosphoramidites, controlled-pore glass columns and all synthesis reagents were obtained from Applied Biosystems, Foster City, California.
   Synthetic oligonucleotides were prepared by the phosphite triester method with an Applied Biosystems Model 380A DNA synthesizer using a 10-fold excess of protected phosphoramidites and 1 µmole of nucleotide bound to the synthesis support column. The chemistries used for synthesis are the standard protocols recommended for use with the synthesizer and have been described (Matteucci et al., J. Amer. Chem. Soc., 103:3185-3319 (1981)). Deprotection and cleavage of the oligomers from the solid support were performed according to standard procedures as described by McBride et al., Tetrahedron Letters, 24:245-248 (1983). The repetitive yield of the synthesis as measured by the optical density of the removed protecting group as recommended by Applied Biosystems (1984) was greater than 97.5%.
   The crude oligonucleotide mixture was purified by preparative gel electrophoresis as described by the Applied Biosystems protocols of November 9, 1984 (User Bulletin No. 13). The acrylamide gel concentration varied from 10 to 20% depending upon the length of the oligomer. The purified oligomer was identified by UV shadowing, excised from the gel and extracted by the crush and soak procedure (Smith, Methods in Enzymology, 65:371-379 (1980)).
2. Sequencing of DNA. DNA sequences were determined by the following methods. Fragments containing the region of interest were cloned into the multiple cloning site of M13mp18 or M13mp19 (Maniatis et al., 1982, and Norrander et al., 1983). Single-stranded DNA was prepared and sequenced by the primer extension method (Sanger et al., 1977 and Biggin et al., 1983) using 35S-deoxyadenosine 5'-(alpha-thio)-triphosphate (New England Nuclear) as label. In some cases, reverse transcriptase (Molecular Genetics) was used to extend the primer, using the dideoxy:deoxynucleosidetri-phosphate ratios utilized by Zagursky et al. (Gene Anal. Tech. (1985) 2:89-94). Deoxyadenosine triphosphate labeled with either ³²P or ³⁵S was used in these reactions. Compression artifacts which appeared in some G-C rich sequences were overcome by eliminating deoxyguanosine triphosphate from the G reaction, and using deoxyinosine triphosphate (P-L Biochemicals) at a final concentration of 37.5 µM instead. In the other mixes, the concentration of dideoxyGTP in the G reaction was 0.5 mM. All sequences were run on 6 or 8% polyacrylamide gels containing 8 M urea (Sanger et al. 1978). Primers used for sequencing were purchased from P-L Biochemicals. Storage and analysis of data utilized software from both DNAstar and International Biotechnologies, Inc.

### Fermentation Conditions

The fermentor is a 15 L Chemap, 10 L working volume. The culture conditions are: temperature = 30°C, pH = 6.8; NaOH 2.5 M is used for pH regulation. The headspace pressure is below 0.1 bar. The dissolved oxygen is regulated at 50%. The air flow varies from 0.5 L/min to 20 L/min. The agitation rate varies between 200 to 1500 rpm.

The fermentor is inoculated with a 10% (v/v) inoculum grown in medium A for 15 hours at 30°C under agitation.

Medium B was the fermentor medium. The starting volume was 5 L.

When the glucose concentration reached 1%, a concentrated solution (5x) of medium B was added to the fermentor in order to keep the glucose concentration approximately at 1%. When the culture reached an OD₆₀₀ of 60.0, the temperature was increased to 42°C for 10 min, then lowered to 39°C for 2.5 hours. The cells were then harvested by centrifugation and frozen at -70°C until processed.

**Table 1**

| Constituent | g/L |
|---|---|
| Medium A: LB Medium | |
| NaCl | 10 |
| tryptone | 10 |
| yeast extract | 5 |
| kanamycin | 5x10⁻³ |
| | |

| Medium B | |
|---|---|
| NH₄Cl | 4.5 |
| KH₂PO₄ | 0.76 |
| MgSO₄·7H₂O | 0.18 |
| K₂SO₄ | 0.09 |
| CaCl₂ | 24x10⁻³ |
| FeSO₄·7H₂O | 7.6x10⁻³ |
| Trace Elements | 0.5 ml |
| casamino acids | 25 |
| yeast extract | 5 |
| glucose | 20 |
| kanamycin | 5x10⁻³ |

### Example 2

### Assembly and Expression of the SLPIII Gene

1. Summary of the scheme for assembling the SLPIII gene. SLPIII closely resembles the silk fibroin molecule because it includes the amino acid tyrosine at regular intervals (about 60 residues). The SLPIII gene was assembled from smaller parts. First, three double-stranded sections of DNA of about 60 bp in length were chemically synthesized. Each section was cloned by insertion into bacteriophage M13 and the DNA sequence was verified. These sections were then removed from the vector and linked together in a specific order. This linkage of about 180 bp is named the SLPIII "monomer". "Monomers" were then linked in a specific order to yield dimers, trimers, tetramers, etc., of SLPIII. The multimers were then cloned either directly into plasmid expression vectors to detect the SLPIII protein or initially into an adapter plasmid. Insertion of the SLPIII DNA into the adapter allows for further gene manipulation and is further described later. The assembly scheme is pictured as follows: 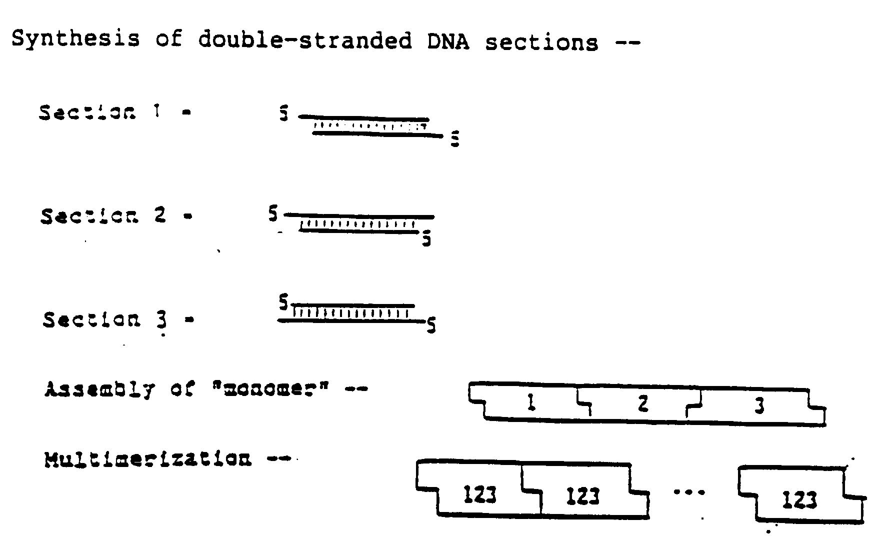
Synthesis of double-stranded DNA sections --

Section 1 - 5 5

Section 2 - 5 5

Section 3 - 5 5

Assembly of "monomer" -- 1 2 3

Multimerization -- 123 123 ... 123
   The DNA and corresponding amino acid sequences of the three sections of the SLPIII gene are shown in Table 2. 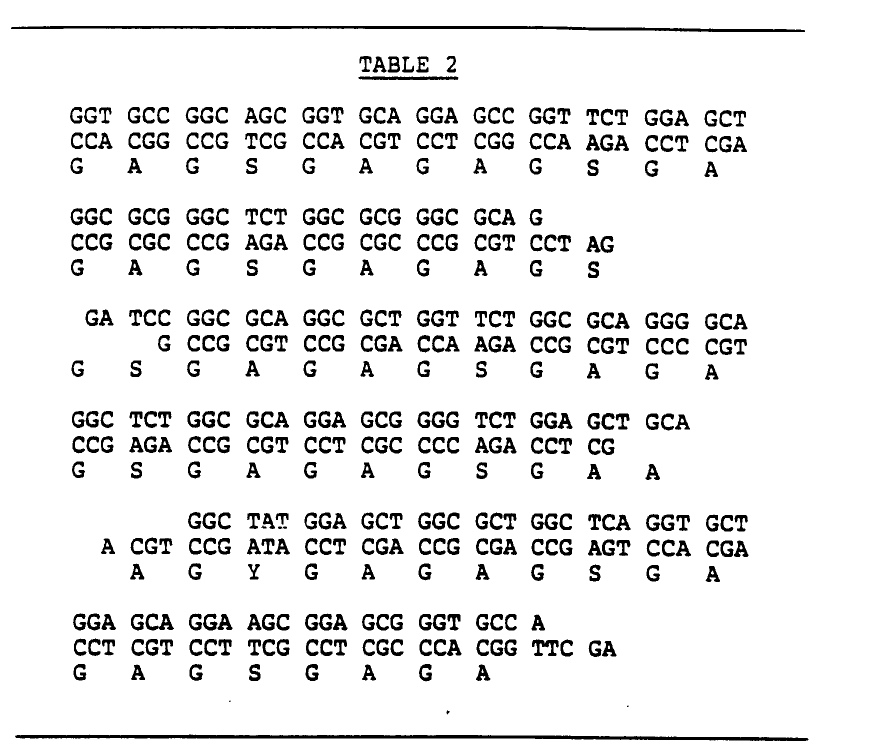
   The double-stranded DNA sequence is shown in the 5' to 3' direction. The amino acids (g = glycine, a = alanine, s = serine, y = tyrosine, coded by the sequence are shown immediately below each section.
   The above six single-strands were synthesized. After synthesis, the strands of DNA were purified and the homologous strands were annealed. About 1 µl (0.5 µg) of each strand was mixed with 2 µl of 10X AA (10XAA defined in Example 1, section 8), buffer and 16 µl of sterilized deionized H₂O in a 1.5 ml polypropylene Eppendorf tube. The tube was placed in a boiling water bath (500 ml in a 1 liter beaker) for 10 min and then the beaker was removed from the hot plate and allowed to cool on the bench to room temperature. This required about 1-2 hr.
   Each of the three double-stranded sections was cloned separately into M13mp18. Section 1 was ligated between the SmaI and BamHI restriction sites of the multiple-cloning site. Section 2 was ligated between the BamHI and PstI sites. And section 3 was inserted between the PstI and HindIII sites. The respective clones are: M13mp18.1, M13mp18.2, M13mp18.3. The DNA sequence was determined for each cloned section. One representative of each section that had the correct DNA sequence was recovered and became the material for the next step: assembly of the "monomer".
2. Assembly of the "monomer" of SLPIII. The DNA sections 2 and 3 were isolated by digestion of the M13 clones with restriction enzymes: For section 2, M13mp18.2 was digested with BamHI and Pstl; for section 3, M13mp18.3 was digested with Pstl and HindIII. The two sections were purified and mixed together in equal molar amounts with M13mp18.1 that had been first digested with BamHI and HindIII. T₄ DNA ligase was added to link the homologous overlapping ends in the order 1-2-3. Due to the hybridization specificity of the cohesive ends, the three sections are efficiently linked in only this order. The DNA sequence of the cloned "monomer" in the assembly named M13mp18.1.2.3 was determined to be correct and as shown in 2 above.
3. Multimerization of the "monomer" of SLPIII. In order to prepare large amounts of the "monomer" structural gene we first subcloned the "monomer" into the plasmid vector pUC12. M13mp18.1.2.3 was digested with EcoRI and HindIII restriction enzymes. The SLPIII "monomer" was gel purified and ligated into pUC12 digested with EcoRI and HindIII. The resulting plasmid DNA was prepared, the "monomer" was released from the vector by digestion with BanI REN and the fragment was gel purified.
   To create multimers, "monomer" DNA with BanI ends were linked by ligation. The nonpalindromic terminal BanI recognition sequence allows linkage only in a head-to-tail order. The extent of multimerization is monitored by gel electrophoresis and staining the DNA with ethidium bromide. Multimers of more than 20 units were obtained by this method.
4. Cloning of the multimers of SLPIII. Plasmid pCQV2 (Queen et al., J. Appl. Mol. Gen., 2:1-10 (1983)) was digested with EcoRI and BamHI restriction endonucleases and a fragment of about 900 bp was purified. This DNA fragment contains the bacteriophage lambda cI-857 repressor gene, the closely linked rightward promoter, P_{R}, and the beginning of the cro gene. Plasmid pSY335 (described as pJF751 in Ferrari et al., J. Bacteriology, 161:556-562 (1985)) was digested with EcoRI and BamHI restriction enzymes and subsequently ligated to the DNA fragment of approximately 900 bp of pCQV2. The plasmid obtained from this construction, pSY751, expresses the β-galactosidase gene at 37°C and 42°C, but not at 30°C.

In this approach the SLPIII gene is first cloned into an "adapter" sequence in an intermediate plasmid and then subcloned to the expression systems. The adapter sequence has the following useful features: a unique central BanI REN site, three unique REN sites to either side of BanI, information coding for protein cleavage at either methionine, aspartateproline or arginine amino acids and small size. The BanI site is the point of insertion for the SLPIII multimers with BanI ends.

The adapter was synthesized with the Applied Biosystems 380A Synthesizer, cloned in M13mp18 and the DNA sequence verified. The adapter was then subcloned into a specially-constructed plasmid vector that lacked BanI REN sites. The recipient plasmid was made as follows. Plasmid pJH101 (Ferrari et al., 1983) was partially digested with AhaIII restriction enzyme and religated. Transformants of E. coli HB101 were selected on medium containing chloramphenicol (12.5 mg/ml). After restriction analysis of several isolates one plasmid was chosen, pSY325. This plasmid contains only the chloramphenicol-resistance gene and the replication origin (from pBR322) of pJH101. After digestion to completion with XhoII, pSY325 was ligated with the gel-purified adapter. The result was the adapter-plasmid, pSY937, and its new REN sites were verified.

The SLPIII multimers were cloned into the BanI site of pSY937. Positive clones were identified by colony hybridization and with the lower strand of section 1 of SLPIII as the DNA probe for hybridization (probe sequence shown in Table 2). Positive clones were characterized by gel electrophoresis for the size of the inserted multimer. Finally, the SLPIII sequences were subcloned using the REN site in the flanking adapter regions to specific locations of expression plasmids.

The SLPIII protein-had the following amino acid composition:

SLPIII 1178 AA MW 83,000

(fm) DPVVLQRRDWENPGVTQLNRLAAHPPFASDPM
GAGS (GAGAGS)6 GAAGY
[(GAGAGS)9 GAAGY]18
GAGAGSGAGAGSGAGAMDPGRYQLSAGRYHYQLVWCQK

(fm) intends the initiation codon

### SLPIII Expression Vector

Plasmid DNA pSyl086 is a pSY937 derivative containing 19 repeats of SLPIII (3.5 kb). This plasmid DNA was digested with NruI and PvuII and the fragments separated by agarose gel electrophoresis. The purified SLPIII multimer was then cloned in plasmid pSY751 digested with PvuII REN. Several clones were analyzed and one (pSyl008) was chosen to be used in expression experiments and SLPIII purification.

The ampicillin drug resistance gene of pSyl008 was substituted with the Kanamycin marker from pSyl010 (produced by digestion of pSY633 with DraI and SspI and insertion of Kan^{R} obtained by HincII digestion of pUC4K) and the subsequent plasmid was called pSY1186. By removing the SLPIII portion of plasmid pSY1186 with BanI, a new plasmid, pSyl262, was generated. This plasmid contains a unique BanI site which allows for the direct ligation of fragments containing BanI ends obtained by polymerization of monomers. This plasmid has been used to generate plasmids containing inserts for the following proteins: SELP1, 2, 3, and SLP4.

### Production and Purification of SLPIII

Cell Culture. E. coli are cultured in the following medium:

| Constituent | g/l |
|---|---|
| | |
| yeast extract | 20 |
| casamino acids | 20 |
| peptone | 20 |
| gelatin peptone | 20 |
| KH₂PO₄ | 2 |
| K₂HPO₄ | 2 |
| Na₂HPO₄ · 7H₂O | 2 |
| glucose | 2 |
| ampicillin | 0.1 |

An overnight culture (500 ml - 1 L) which had been grown at 30°C was used to inoculate 375 L of media contained in a 500 L fermentor. Fermentor conditions include a tachometer reading of 100 rpm, vessel back pressure of 5 psi and an air flow of 170 l/min in order to maintain dissolved O₂ at greater than 50%.

Glucose (1 gm/l) and ampicillin (0.05 g/l) were added to the fermentation when the culture reached an OD₆₅₀ of 1.0 and again at 2.0. When the culture reached an OD₆₅₀ of 2.0 the temperature was increased to 42°C for 10 min and then lowered to 38°C for 2 hours. The culture was then chilled to 10°C and cells were harvested by centrifugation in a continuous centrifuge and frozen at -70°C until processed. Yields from two separate fermentations were 7.3 kg and 5.2 kg wet weight of cells.

It should be noted that other media can be used and, with different plasmids, various selection conditions can be imposed (i.e., substitution of kanamycin selection for ampicillin). These conditions have been used in laboratory scale fermentations (10 L volumes).

### Cell Lysis.

Method 1. Cells are thawed and suspended to a concentration of 1 kg wet weight/6 1 in 50 mM TRIS-HCl pH 7.0, 1 mM EDTA and broken by 2 passages through an APV Gaulin cell disrupter at 8000 psi. During this lysis procedure the cells are kept cold with an ice bath. The cell lysate is then centrifuged at 26,000xg with a continuous centrifuge, such as the TZ-28 rotor in a Sorvall RCSB refrigerated centrifuge operated at 4°C. Under these conditions greater than 90% of the SLPIII produced can be found in the pellet. The supernate does contain some product which can be recovered by NH₄SO₄ precipitation as described below. The pellet is extracted with LiBr as described below.
Method 2. Frozen cells are thawed and resuspended to a concentration of 1 kg wet weight/6 1 in 50 mM TRIS-HCl pH 7.0, 10 mM EDTA, and 5 mM PMSF to inhibit protease activity. Cells are stirred in this buffer at room temperature for 0.5 to 2 hours, then lysozyme is added to a concentration of 1 g/l and incubation is continued for 20 min. β-Mercaptoethanol is then added to 70 mM and the detergent NP40 is then added to a final concentration of 1% for 20 min while continuously stirring the cell suspension. Then MgCl₂ is added to 50 mM followed by DNAse at a concentration of 1 mg/l and incubation is continued at room temperature for 20 min. The cell lysate is then centrifuged as in method 1 at 26,000xg in a continuous centrifuge and the supernatant is collected and passed through the continuous centrifuge a second time at 26,000xg. The supernate resulting from this second centrifugation contains <5% of the total SLPIII, but what is there can be recovered with NH₄SO₄ as described below. The pellets resulting from the 1st and 2nd 26,000xg centrifugations are combined and extracted with LiBr as described below.
Method 3. For this method, a strain of E. coli is used that contains a second plasmid which encodes the T7 phage lysozyme. This plasmid is compatible with the plasmid encoding the SLPIII gene and the drug resistance determinant. The strain is grown in the same medium and under the same conditions as in the first two methods. However, due to the production of the T7 lysozyme inside the cells, their cell wall is weakened and they can be easily lysed at the completion of the fermentation by the addition of EDTA to >100 mM and NP40 to a concentration of from 0.5 to 1.0% v/v. Lysis can also be achieved by the addition of chloroform (20 ml per liter) of fermentation broth instead of NP40. Alternatively, cells may be collected by centrifugation prior to lysis, resuspended to 1 kg wet weight/6 1 in TRIS-EDTA as described in the first two methods and then lysed by the addition of NP40 or chloroform. Following cell lysis by either method the lysate is centrifuged in a continuous rotor at 26,000xg as described in the first two methods. As with those methods, LiBr extraction of the pellet and NH₄SO₄ precipitation of the supernate are used to recover the product.

### Purification of SLPIII

The pellet obtained by centrifugation of the cell lysate at 26,000xg as described above is extracted with an equal volume of 9M LiBr. The salt solution is added and the pellet is evenly suspended by stirring at room temperature (RT). The mixture is stirred for 1 hour at RT after an even suspension is obtained. The mixture is then centrifuged at 26,000xg in a continuous rotor at 4°C or at RT to generate a pellet and a supernatant fraction. The supernate is saved and the pellet is re-extracted with another equal volume of 9 M LiBr as above. After mixing for 1 hour the mixture is centrifuged at 26,000xg and the supernate from this centrifugation is combined with the supernate from the first LiBr extraction and allowed to stand at 4°C overnight. Approximately 90% of the SLPIII contained in the cell lysate 26,000xg pellet is extracted by LiBr using this procedure.

After the LiBr extract stands overnight at 4°C a precipitate forms, is removed by centrifugation at 26,000xg and is discarded. The supernate is then placed in dialysis bags and dialyzed against several changes of dH₂O for 2 days. As the LiBr is removed by dialysis the SLPIII product precipitates in the dialysis bags. The precipitate is collected by centrifugation and washed 2 to 3 times with dH₂O. The final washed product is centrifuged and dried by lyophilization.

For the recovery of SLPIII from the 26,000 g supernatant fractions, NH₄SO₄ precipitation is used. Solid NH₄SO₄ is slowly added to the sample which is maintained at 4°C, until 38% saturation is achieved (231 g/l). The mixture is then stirred at 4°C for 2 to 3 hours. The precipitate is recovered by centrifugation in a continuous flow centrifuge and washed 4 to 5 times with an equal volume of distilled H₂O or with 0.5% SDS in H₂O. After each wash the precipitate is recovered by continuous centrifugation. The pellet becomes increasingly white with successive washes as contaminating protein is removed. SLPIII is recovered as a washed pellet and can be dried by lyophilization.

### Trypsin Treatment Step of SLPIII

SLPIII was suspended in 50 mM TRIS-HCl, pH 8.0, 0.1 M NaCl buffer, and was placed in a 37°C water bath, and TPCK treated trypsin solution was mixed into the suspension. The final trypsin concentration was 0.1%. After 3 hours, the solution was centrifuged at 16,000xg for 15 min., the pellet was washed with a half equal volume of 0.5% SDS in H₂O first, then with distilled water. After each wash the pellet was recovered by centrifugation. The final product was resuspended in water and kept at 4°C for further analysis.

With the trypsin treatment, SLPIII was purified to 99.4% purity.

### Physical Measurements of SLPIII

Physical measurements of the purified silk-like proteins have been compared with those of Bombyx mori silk in order to establish that the repetitive amino acid polymers produced microbiologically accurately mimic the properties of naturally occurring polymers. Physical measurements were performed to confirm the model of anti-parallel chain pleated sheet conformation for the crystalline regions of Bombyx mori silk fibroin (Marsh, Corey and Pauling, Biochem. Biophys. Acta (1955) 16; Pauling and Corey, Proc. Natl. Acad. Sci. USA (1953) 39:247. Preliminary analysis of x-ray difraction patterns obtained from SLP films are consistent with those described by Fraser, MacRai, and Steward (1966) (Table 3). Circular Dichroic (CD) and Fourier transform infrared (FTIR) spectroscopic analysis of SLPIII are consistent with a high degree of extended β and β-turn conformations. Comparisons of the spectra obtained from SLPIII with that of naturally occurring silk fibroin in various solvents (Isuka and Young, Proc. Natl. Acad. Sci. USA (1966) 55:1175) indicate that SLPIII in solution consists of a mixture of the random and highly ordered structures seen in silk fibroins.

This data demonstrates that SLPIII contains extended β strands that are capable of packing into highly crystalline domains. The data also reveals the presence of distinct 8-turn (reverse turn) conformations. A computer analysis of the amino acid sequence of SLPIII using an algorithm for secondary structure prediction developed by Chou and Fasman (1974), infra, indicates that the AGYG sequence has a β-turn propensity.

**Table 3**

| Material | a (A) | b (A) | c (A) |
|---|---|---|---|
| (AG)ₙ | 9.42 | 6.95 | 8.87 |
| (AGAGSG)ₙ | 9.39 | 6.85 | 9.05 |
| CTP fraction | 9.38 | 6.87 | 9.13 |
| Native fibroin | 9.40 | 6.97 | 9.20 |
| | | | |
| SLPIII | 9.38 | 6.94 | 8.97 |
| Referenced in Fraser et al., J. Mol. Biol. (1966) 19:580. | | | |

### Example 3

### FCB-SLPIII Construction

### Design

The SLPIII polymer (see above and also Application Serial No. 114,618, filed October 29, 1987, PCT/US/87/02822) was chosen because of its predicted structure, allowing for fabrication of useful products; having good structural properties for use in a wide variety of applications; having 8-turn structures between interactive strands; and allowing for substitution of the turn sequences with other sequences. The fibronectin cell-binding domain, amino acids 1405-1512, has a strong turn propensity, with the tripeptide RGD providing for cell attachment, predicted to be present within a hydrophilic loop between adjacent β-strands. A 10 amino acid sequence spanning this proposed loop structure (referred to as fibronectin cell-binding or FCB sequence) was chosen to constitute the functional block of amino acids to be inserted within the SLPIII backbone. The insertion site within the SLPIII backbone was chosen to correspond with the amino-acid sequence GAAGY which is also predicted to provide a turn structure (Chou and Fassman (1974) Biochemistry 13:222-244). The design allows for conservation of the FCB structure while causing minimal disruption of the SLPIII (GAGAGS)₉ β-strand crystal-packing domains.

### DNA Synthesis and Gene Construction

The SLPIII gene monomer contains a PstI restriction endonuclease site within the sequence encoding the proposed turn structure, GAAGY. This site was used to insert the synthetic DNA encoding the 10 amino acids of the FCB sequence. Two complementary DNA strands comprising the FCB site, 36 bases in length, were synthesized consisting of the sequence shown below:

5'-GTGACTGGCCGTGGTGATAGCCCGGCTAGCGCTGCA-3'
3'-ACGTCACTGACCGGCACCACTATCGGGCCGATCGCG-5'

These oligonucleotides were purified according to the procedures described in Example 1, and cloned into the PstI site of pSyl304. pSyl304 contains a single monomer gene fragment of SLPIII. pSyl304 DNA was digested with PstI and ligated with a mixture of the FCB oligonucleotides. The ligation reaction products were transformed into E. coli cells. Colonies containing the plasmid were selected on bacterial culture plates containing the antibiotic chloramphenicol. Individual colonies were grown and plasmid DNA purified and analyzed for the presence of the FCB oligonucleotide sequence by restriction digestion with NheI. Plasmids containing this restriction site were subjected to DNA sequencing and two candidates were shown to be correct. The partial nucleotide sequence of one of these, pSyl325, and the encoded amino-acid sequence is shown as follows:

Figure 3

1 15 30
Ban I
1
GGT GCC GGC AGC GGT GCA GGA GCC GGT TCT GGA GCT GGC
A G S G A G A G S G A G
5 10

45 60 75
Bam HI
1
GCG GGC TCT GGC GCG GGC GCA GGA TCC GGC GCA GGC GCT
A G S G A G A G S G A G A
15 20 25

90 105

GGT TCT GGC GCA GGG GCA GGC TCT GGC GCA GGA GCG GGG
G S G A G A G S G A G A G
30 35

120 135 150
Pst I
1
TCT GGA GCT GCA GTG ACT GGC CGT GGT GAT AGC CCG GCT
S G A A V T G R G D S P A
40 45 50

165 180
PST I
1
AGC GCT GCA GGC TAT GGA GCT GGC GCT GGC TCA GGT GCT
S A A G Y G A G A G S G A
55 60

195 210
Ban I
1
GGA GCA GGA AGC GGA GCG GGT GCC
G A G S G A G
65 70

The FCB-SLP monomer gene fragment was purified from pSyl325 by digestion with BanI, agarose-gel electrophoresis, and NACS purification (Example 1, No. 11). The monomer gene fragment was self-ligated and cloned into pSY937 which had been digested with BanI. The products of this ligation were transformed into E. coli and selected for growth on chloramphenicol. Plasmid DNA from individual colonies was analyzed for inserts containing multiple FCB-SLP monomer fragments by digestion with NruI and EcoRV and electrophoresis on agarose gels. One clone was identified containing two inserts, one of approximately 2.1 kb and the other of 2.8 kb. Both inserts were cloned individually and transferred to the expression vector pSY751. Plasmid pSyl325 was digested with NruI and PvuII and the 2.1 and 2.8 kb insert bands were purified. These DNA fragments were ligated with pSY751 that had been digested with PvuII. The products of this reaction were transformed into E. coli and selected for growth on the antibiotic ampicillin. Plasmid DNA from individual colonies was analyzed by restriction digestion for the presence of the FCB-SLP polymer gene. Two clones were identified, pSyl520 and 1521, containing the 2.1 and the 2.8 kb inserts, respectively.

### Product Purification

E. coli cells containing pSyl520 and pSyl521 were grown at 30°C in LB medium containing 50 µg/ml ampicillin to an OD₆₀₀ of 0.7. Production of the FCB-SLP polymer proteins were induced by increasing the culture temperature to 42°C for 1.5 hrs. The cells were harvested by centrifugation and lysed in sample buffer containing sodium dodecylsulfate (SDS) and β-mercaptoethanol by heating at 100°C for 5 min. Samples of these lysates corresponding to 5 x 10⁸ cells were applied to an 8% polyacrylamide gel containing SDS, electrophoresed, and transferred to nitrocellulose filters by electroblotting. The filters were incubated either with anti-SLP or anti-FCB peptide antibody. Specific immunoreactivity with the anti-SLP antibody was observed for a protein band of approximately 75 kd in lysates of pSyl520, 95 kd in lysates of pSyl521, and 120 kd in lysates of the SLPIII clone pSY1186. Reactivity with the anti-FCB antibody was observed only for the two FCB-SLP polymer bands.

E. coli containing pSyl521 was fermented at the 1 L level using the batch process conditions described in Example 1. 25 g of wet-weight cells were harvested by centrifugation and disrupted by French pressing at 15,000 psi. The cell lysate was centrifuged at 16,000xg for 20 min, yielding a pellet fraction containing >90% of the FCB-SLP protein as determined immunologically. The pellet was washed several times with 50 mM TRIS-HCl (pH 8.0), 150 mM NaCl, 1 mM EDTA and then extracted with 5 M LiBr. The supernatant phase of this extraction was dialyzed against deionized water resulting in the formation of a precipitate which was collected by centrifugation. The precipitate was re-extracted with 3 M LiBr and the dissolved proteins were selectively precipitated by 4-fold dilution with distilled water. The remaining supernatant was dialyzed against 10 mM TRIS (pH 7.5), 1 mM EDTA, 1 mM PMSF. The precipitated protein was collected by centrifugation, resuspended in deionized water, and analyzed by amino-acid composition. The composition of this semi-purified fraction showed it to be approximately 30% FCB-SLP as determined by compositional content of the amino acids present in this polymer. The ratio of these amino acids correlated closely with that predicted by the DNA sequence of the FCB-SLP polymer gene.

### Assay of Biological Activity

In order to determine whether the FCB-SLP polymer protein exhibited cell attachment activity, semi-purified protein was immobilized on nitrocellulose filters and incubated with mammalian tissue-culture cells. Lysate proteins from E. coli cells containing pSyl520, pSyl521, or pSyl186 (the SLPIII-encoding plasmid) were electrophoresed on SDS-PAGE and transferred to nitrocellulose filters by electroblotting. These filters were tested for their ability to promote cell attachment using a modification of the procedure of Hayman et al. (1982) supra. The filters were washed in PBS (10 mM Na phosphate (pH 7.4), 150 mM NaCl) several times with gentle agitation, and soaked in PBS containing 5% condensed milk for 1 hr. The filters were then either incubated with 5% condensed milk in PBS containing anti-SLP or anti-FCB serum (1:100 dilution of serum) or 5% condensed milk in PBS alone for 2 hr at 37°C. The filters were washed twice with PBS and incubated with cells. The cells were prepared by resuspension of subconfluent monolayers in 0.1% trypsin solution. The cells were incubated in DME medium containing 10% fetal calf serum at 0°C for 30 min. The cells were centrifuged (except for H9 lymphocytes which were allowed to settle), washed in PBS, and finally resuspended in DME medium without serum at a density of 2.5 x 10⁶ cells/ml. The filters were overlayed with the cell suspensions in flat plastic dishes and incubated at 37°C in a CO₂ incubator for 1 hr. The filters were washed twice in PBS to remove unbound cells and fixed in 3% formaldehyde. The filters were stained with Amido black in 45% methanol, 10% acetic acid, 45% water for 3 min and destained in 90% methanol, 2% acetic, 8% water.

By visual inspection, the filter incubated with Vero cells (African green monkey kidney epithelial cells) contained heavily stained regions. These regions corresponded to the positions of the FCB-SLP protein bands as determined by reactivity to parallel filters with anti-SLP and anti-FCB antibody. Microscopic inspection of these same areas of the filters showed that the heavy staining was due to the attachment of cells. No cells above background were bound to any other area of the filters including other E. coli lysate protein bands. Cells were not attached to the filter at the region corresponding to the SLPIII protein band. As expected, when incubated with H9 lymphocytes, no specific binding of cells to the filter was observed.

In order to determine the effect of protein concentration and various pretreatments of filters and cells with specific antibodies on cell attachment, a dot-blot cell-binding assay was developed. The assay was performed as described above except that semi-purified FCB-SLP and SLPIII protein of known concentration was applied directly to nitrocellulose filter dots using a Schleicher and Schuell dot-blot manifold. Each sample was applied such that the first dot in a series contained 10 µg of protein and subsequent dots contained serial two-fold dilutions of this sample. Both the 75 kd and the 95 kd FCB-SLP proteins from pSyl520 and 1521, respectively, promoted Vero cell attachment at low concentration. The minimum amount of the 75 kd FCB-SLP protein required to promote cell attachment was 0.05 µg/cm². For the 95 kd FCB-SLP protein, the minimum was 0.2 µg/cm². Few cells were attached to the SLPIII-containing dots even at 12.5 µg/cm². Cell attachment to the FCB-SLP protein was inhibited by preincubation of the filter with anti-FCB or anti-SLP antibody. Cell attachment was inhibited but not abolished by preincubation of the cells with FCB peptide at a concentration of 300 µg/ml.

The introduction of the 10 amino acids of FCB into the SLPIII monomer sequence creates a protein polymer whose purification and solubility characteristics are not appreciably different from those of the SLPIII protein polymer, while gaining the additional property of the ability to promote cell attachment. The subject protein polymers may be formulated, either singly or in combination, with other compounds or protein polymers, synthetic or natural polymers, into fibers and films that promote cell attachment. The biological activity of FCB-SLP is chemically resistant to denaturation. Cell attachment occurs to the FCB-SLP proteins after treatment with high concentrations of detergent at 100°C, as well as with chaotropic denaturants. This stability facilitates the sterilization methods used in the preparation of bioactive materials. Solid-support cell-attachment matrices for enhancing the growth and differentiation of cells and tissue culture is a direct application of such materials. The subject polymers may be easily deposited onto other materials or substrates to provide for a cell-binding surface. Cell-attachment polymers in various forms, e.g., fibers, membranes, films, etc. may be used in bioreactors to suspend production cells within gently mixing liquid growth medium, increasing exposure to nutrients while decreasing cell damage to vigorous agitation. The subject material may be made into or coated on woven fabrics, films or membranes and used as wound-dressings that promote enhanced healing due to the attachment of cells involved in the healing process. In addition, the subject proteins may be formulated into or deposited onto the walls of prosthetic devices intended for in vivo replacement of blood vessels, bone joints, tendons, ligaments, or the like, in order to promote in vitro and/or in vivo colonization of the device, thus improving its functional properties. In addition, the subject proteins may find application in the eye, as implants, carriers, coatings or the like.

### SLP3-Cys (SLP-C)

### Design of a chemically active cross-linkable polymer.

Many amino acids contained within a protein chain can be chemically modified using specific organic and inorganic reagents. Some of these amino acids include lysine (K), arginine (R), glutamate (E), aspartate (D), cysteine (C), serine (S), threonine (T), histidine (H), and tyrosine (Y). In order to create a protein polymer upon which specific reactions could be performed for the purpose of adding additional molecules to the chain at specific locations or to link chains of the same or different polymers together, or to link chains to a suitable surface, SLP-C was designed. The 59 amino acid silk-like monomer of SLP3 was modified to include the amino acid sequence GAGCGDPGKGCCVA. Features of the inclusion sequence are: 1. the GAGC and GCCV tetrapeptides conform with the B-strand structure of the flanking silk-like amino acids and contain cysteines whose sulfhydryls are reactive under oxidation and can be covalently crosslinked, 2. the DPGK does not conform with the B-strand structure therefore forcing an unaligned structure within the silk-like strands. This latter sequence further has the advantage of providing two additional chemically modifiable residues, aspartate and lysine, and contains the dipeptide DP which is susceptible to specific chemical peptide cleavage.

### Polymer Construction.

Two oligonucleotide strands were synthesized and purified as described in the Methods section.

PstI                    SmaI
1)5'-     GGAGCAGGTTGTGGCGATCCCGGGAAAGGATGCTGTGTAGCTGGTGCA-3'
2)3'-ACGTCCTCGTCCAACACCGCTAGGGCCCTTTCCTACGACACATCGACC     -5'

The two oligonucleotide strands were annealed and ligated with the DNA of plasmid pSyl304 (SLP3 monomer in pSY937) which has been digested with PstI REN.

The product of this ligation reaction was transformed into E. coli strain HB101. Plasmid DNA from transformants was purified and digested with BanI; clones containing inserts of the correct size were digested with PstI and EcoV RENs for the determination of orientation. Plasmid DNA from correct clones was sequenced. Plasmid pPTO103 (shown in Table 4) contained the desired SLP-C monomer sequence, oligonucleotides (1) and (2) are shown below in bold and underlined.

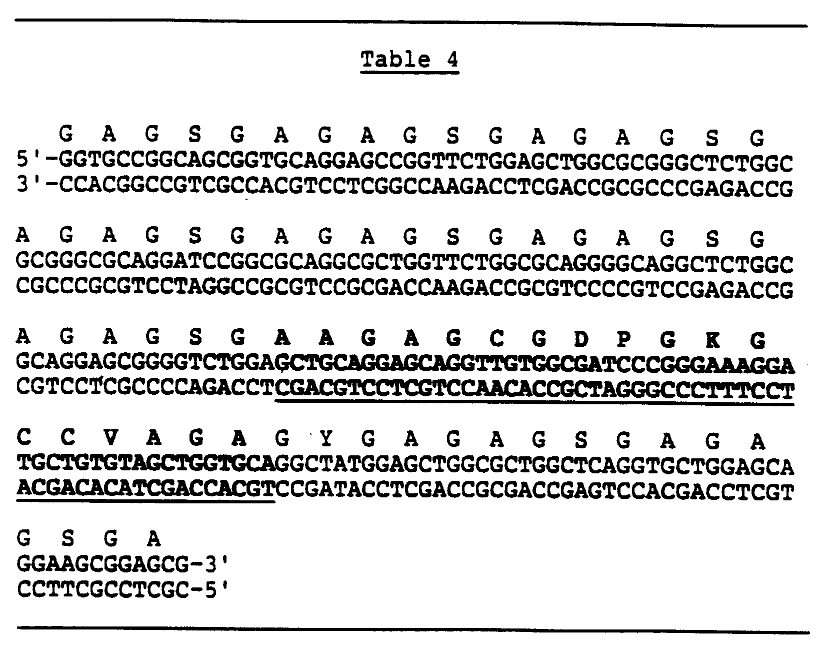

Plasmid DNA from pPT0103 was digested with BanI REN and the digestion fragments were separated by agarose gel electrophoresis. The SLP-C gene fragment, 225 bp, was excised and purified by NACS column (see Methods). The purified fragment was ligated with plasmid pSyl262 which had been digested with REN BanI. The product of this ligation reaction was transformed into E. coli strain HB101. Transformants were selected for resistance to kanamycin. Plasmid DNA from individual transformants was purified and analyzed for increase in size due to SLP-C multiple DNA insertion. Several clones were obtained ranging in size from approximately 1.0 kbp to 5.4 kbp. Six clones (pPT0105 →pPT0110) were chosen to be used for expression of SLP-C.

### Expression.

An overnight culture which had been grown at 30°C was used to inoculate 50 ml of media contained in a 250 ml flask. Kanamycin was added at a final concentration of 50 µg per ml and the culture was incubated with agitation (200 rpm) at 30°C. When the culture reached an OD₆₀₀ of 0.8, 40 ml were transferred to a new flask prewarmed at 42°C and incubated at the same temperature for approximately 2 hours. The cultures (30°C and 42°C) were chilled on ice and OD₆₀₀ was taken. Cells were collected by centrifugation divided in 1.0 OD₆₀₀ aliquot and used to perform dot blot and western analysis using SLP antibodies (see Methods section). For purification and amino acids analysis, larger cultures were used.

### Analysis.

E. coli strain HB101 containing plasmid pPT0105 was grown at 30°C to an OD₆₀₀ of 0.7 and then shifted to 42°C, after the addition of 40 µCi per ml of ³⁵S-Cysteine, for 2.0 hours. The proteins produced by these cells were analysed by SDS-PAGE for detection of ³⁵S-Cysteine and reactivity to SLP antibodies (see Methods). In both analyses a strong reactive band was observed with an apparent molecular weight of approximately 150 kD.

pPT0105 SLP-C Protein 1333AA MW97,077
MDPVVLQRRDWENPGVTQLNRLAAHPPFASDPMGAGS(GAGAGS)6
[GAA(GAGCGDPGKGCC)VAGAGY(GAGAGS)9]16
GAAGAGCGDPGKGCCVAGAGY(GAGAGS)2GAGAMDPGRYQLSAGRYHYQLVWCQKZ

### SLP3-Laminin Cell Binding (SLP-L)

### Design of a biologically active polymer for attachment of nerve cells.

The amino acid sequence of human laminin B1, a protein component of some extracellular basement membranes upon which cells attach and proliferate, contains a pentapeptide, YIGSR, which has been implicated in providing a receptor attachment site for nerve cells. Two silk-like polymers, one containing a 12 and the other a 14 amino acid sequence each including the pentapeptide were designed. In the first polymer designated SLP-L1, the 59 amino acid monomer of SLP3 was interrupted near its tyrosine residue with the inclusion of the sequence YCEPGYIGSRCD. In SLP-L2 the inclusion sequence was LCVSEPGYIGSRCD. Since they do not conform to B-strand structure, both sequences when embedded within the B-strand structure of the silk-like strands should form unaligned loops. SLP-L1 and SLP-L2 are designed to provide a synthetic substrate for the attachment of nerve cells both in culture and in vivo. The formulation of these polymers into nerve conduits for the regeneration of injured nerves is one possible application.

### Construction of SLP-L1.

Two oligonucleotide strands were synthesized and purified as described in the Methods section.

PstI SmaI
i) 5'- GTATGTGAACCCGGGTATATCGGTAGCCGTTGCGATGCA-3'
ii) 3'-ACGTCATACACTTGGGCCCATATAGCCATCGGCAACGCT -5'

These oligonucleotide strands were annealed and ligated with plasmid pSyl304 (see WO 88/03533, page 65) which had been digested with PstI REN.

The product of this ligation reaction was transformed into E. coli strain HB101. Plasmid DNA from transformants was purified and digested with BanI; clones containing inserts of the correct size were digested with PstI and EcoV RENs to determine the correct orientation. Plasmid DNA from correct clones was sequenced. Plasmid pT0120 (shown in Table 5) contained the desired SLP-L1 monomer sequence, oligonucleotides (i) and (ii) are shown below in bold and underlined.

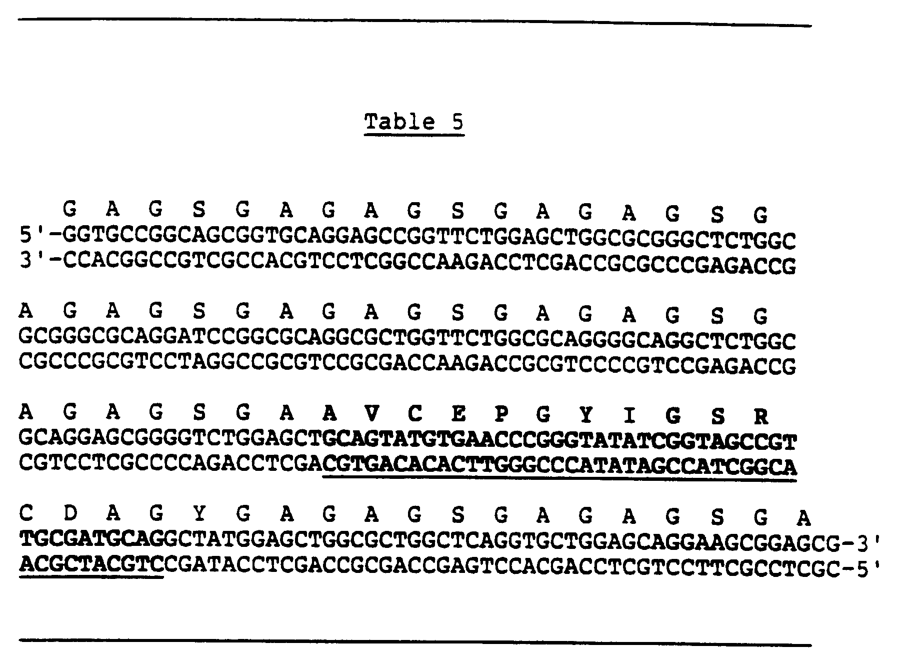

Plasmid DNA from pPT0120 was digested with BanI REN and the digestion fragments were separated by agarose gel electrophoresis. The SLP-L1 gene fragment, 216 bp, was excised and purified by NACS column (see Methods). The purified fragment was ligated with plasmid pSyl262 which had been digested with REN BanI. The product of this ligation reaction was transformed into E. coli strain HB101. Transformants were selected for resistance to kanamycin. Plasmid DNA from individual transformants was purified and analyzed for increase in size due to SLP-L1 multiple DNA insertion. Several clones were obtained ranging in size from 1 kbp to 4 kbp. One clone pPT0123, with an insert of approximately 3.0 kbp was chosen for expression and protein analysis.

### Construction of SLP-L2.

An additional two oligonucleotide strands were synthesized as described in the Methods section.

iii) 5'- CTGTGTGTTAGCGAACCC -3'
iv) 3'- ACGTGACACACAATCGCTTGGG -5'

These oligonucleotide strands were annealed and ligated with plasmid pPT0120 which had been digested with PstI and SmaI RENs. Plasmid DNA from transformant colonies resistant to chloramphenicol was purified. One plasmid, pPT 0121, (see Table 6) which was not digestible with REN PstI, was sequenced and proven to be the desired SLP-L2 monomer gene fragment. The oligonucleotide strands (iii) and (iv) are shown below in bold and underlined.

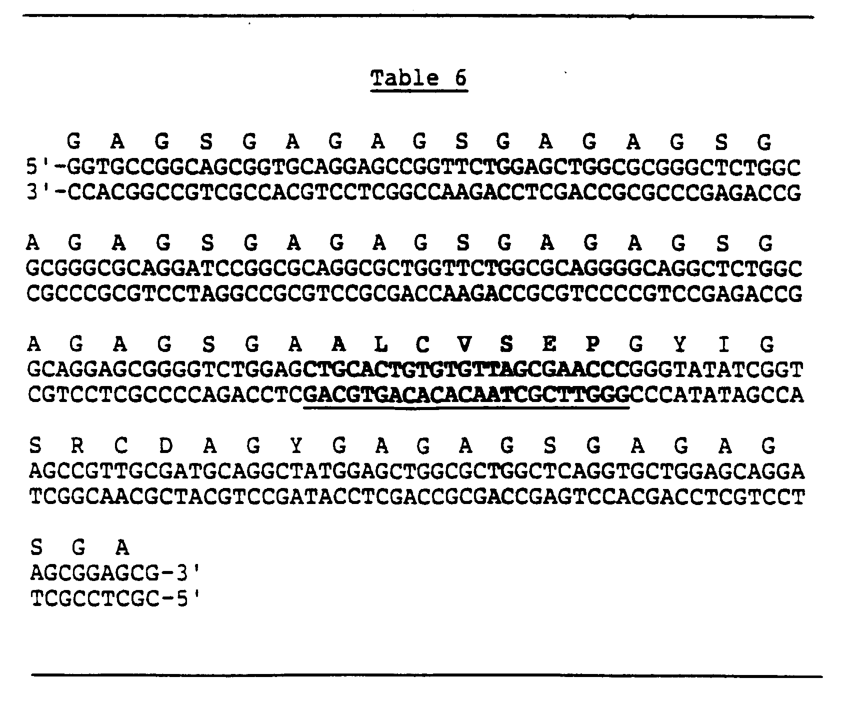

Plasmid DNA from pPTO121 are digested with BanI REN and the digestion fragments are separated by agarose gel electrophoresis. The SLP-L2 gene fragment, 222 bp, is excised and purified by NACS column (see Methods). The purified fragment is ligated with plasmid pSyl262 which has been digested with REN BanI. The product of this ligation reaction is transformed into E. coli strain HB101. Transformants are selected for resistance to kanamycin. Plasmid DNA from individual transformants is purified and analyzed for increase in size due to SLP-L2 multiple DNA insertion. Several clones are obtained ranging in size from 1 kbp to 4 kbp.

### Expression of SLP-1.

An overnight culture which has been grown at 30°C was used to inoculate 50 ml of media contained in a 250 ml flask. Kanamycin was added at a final concentration of 50 µg per ml and the culture was incubated with agitation (200 rpm) at 30°C. When the culture reached an OD₆₀₀ of 0.8, 40 ml were transferred to a new flask prewarmed at 42°C and incubated at the same temperature for approximately 2 hours. The cultures (30°C and 42°C) were chilled on ice and OD₆₀₀ was taken. Cells were collected by centrifugation divided in 1.0 OD₆₀₀ aliquot and used to perform dot blot and western analysis using SLP antibodies (see Methods section). For purification and amino acid analysis larger cultures were used.

pPT0123     SLP-L1 Protein     1066 AA     MW 81,800
MDPVVLQRRDWENPGVTQLNRLAAHPPFASDPMGAGS(GAGAGS)6
[GAAVCEPGYIGSRCDAGY(GAGAGS)9]13
GAAVCEPGYIGSRCDAGY(GAGAGS)2GAGAMDPGRYQLSAGRYHYQLVWCQKZ

SLP-L2 Protein
MDPVVLQRRDWENPGVTQLNRLAAHPPFASDPMGAGS(GAGAGS)6
[GAALCVSEPGYIGSRCDAGY(GAGAGS)9]11
GAALCVSEPGYIGSRCDAGY(GAGAGS)2GAGAMDPGRYQLSAGRYHYQLVWCQKZ

### CLP AND CLP-CB

### The design of a collagen-like polymer with thermoreversible gelation character.

Chemically hydrolyzed natural collagen can be denatured and renatured by heating and cooling to produce gelatin which is used in photographic and medical applications, among other applications. The chain property of collagen responsible for this phenomenon is its ability to spontaneously form interchain aggregates having a conformation designated as a triple helix. The helices are stabilized by weak interactions between chains arising from the close proximity of the peptide backbone at locations every third residue occupied by glycine and kinks provided by proline and hydroxyproline at the two positions between glycines. The geometry of the three kinked chains allows for hydrogen bonding within the triple helix. The structure is loose and is readily accessible to the interaction with water, small organic and inorganic molecules, other proteins, and cells. Although collagen consists of many different amino acid sequences one of the more structurally stable segments exists at the amino and terminal ends of the processed collagen chains. These ends consist almost exclusively of the repeating tripeptide sequence GPP (the second P is often hydroxylated). The collagen-like polymer CLP was designed predominantly of repeating GPP tripeptides (8 within each monomer segment). Other tripeptides were included in order to decrease the overall repetitiveness of the gene and to allow the use of additional codons which could be used to better manipulate the DNA. These were GAP (twice), GPA, GPV, and GSP. All of these triplets occur in natural collagen although never in the sequence and context used in CLP. The properties of CLP were designed to yield a protein polymer that would undergo thermoreversible gelation at high temperatures, as well as being nonimmunogenic. The high stability of the helices should create high tensile strength in fibers or membranes formulated from CLP. These chain properties should allow the creation of hydrogel colloids in aqueous solutions which when applied to hard substrates should act as soft coatings. Because of the simple sequence of CLP, its optical absorbance should be minimal in all wavelengths down to 220 nm.

### The design of a soft coating material with cell attachment function.

The versatile formulation properties of collagen-like polymers makes them ideal as biomaterials used in implantable devices. Either as a coating on the surface of prostheses or as a structural component of the device itself, CLP could promote tissue integration by providing improved blood and cellular interaction. The latter function is mediated in natural collagen by a specific amino acid sequence which acts as a ligand for a cellular receptor. In order to create a CLP polymer with cell attachment activity, the sequence GLPGPKGDRGDAGPKGADGSP was included within the CLP monomer sequence. In contrast to the hydrophobic GPP collagen-like flanking sequences, this block of 21 highly charged hydrophilic amino acids should form a destabilized flexible helical region accessible for interaction with cells. Promotion of epithelialization of the surface of an implanted device will improve the compatibility and rejection characteristics increasing its lifetime and safety. The subject compositions may find use as wound dressings, allowing for neovascularization, eye applications, matrices for artificial organs, and the like.

### Construction of CLP monomer.

The CLP (Collagen-Like Protein) synthetic gene was assembled from smaller parts. First, three double-stranded sections of DNA ranging from 40 to 60 bp in length were chemically synthesized.

EcoRI BanI
A 1)
AATTCGGTGCCCCTGGTCCGCCTGGTCCGCCTGGTCCACCGGGTCCTCCGG
2)
GCCACGGGGACCAGGCGGACCAGGCGGACCAGGTGGCCCAGGAGGCC

BanII (Asp718)
GGGCTC
CCCGAGCATG

BanII SmaI
B 3) CCGGGTCCTCCAGGACCGCCAGGTCCGCCTGGTCCCCC
4) CCGAGGCCCAGGAGGTCCTGGCGGTCCAGGCGGACCAGGGGG

SmaI BanI ↓
C 5) GGGTCCTGCAGGTCCAGTAGGTAGCCCCGGTGCC
6) CCCAGGACGTCCAGGTCATCCATCGGGGCCACGG

FspI EcoRV SalI
ATGTGTGCGCATCGATATC
TACACACGCGTAGCTATAGAGCT

Sections A and C (the sequence of section C after the arrow is not coding for CLP but is a modification of the multiple cloning site of the acceptor plasmid) were cloned separately into plasmid pUC19 that had been digested with the appropriate REN. The DNA sequence was verified and two plasmids were selected, pPT0111 and pPT0112, carrying the correct sequence. Section B was ligated into pPT0111 that had been digested with BanII and SmaI RENs. Plasmid pPT0113 was sequenced and found correct.

Plasmids pPT0112 and pPT0113 were digested with the appropriate RENs to release section C and Sections A + B respectively. After purification these DNA fragments were ligated into pSY937 previously digested with BanI and EcoRV RENs. The product of the ligation reaction was transformed into E. coli strain HB101. Plasmid DNA from transformants was purified and digested with BanI; clones containing inserts of the correct size were sequenced. Plasmid pPT0116 contained the desired sequence shown for section A + B + C (see Table 7).

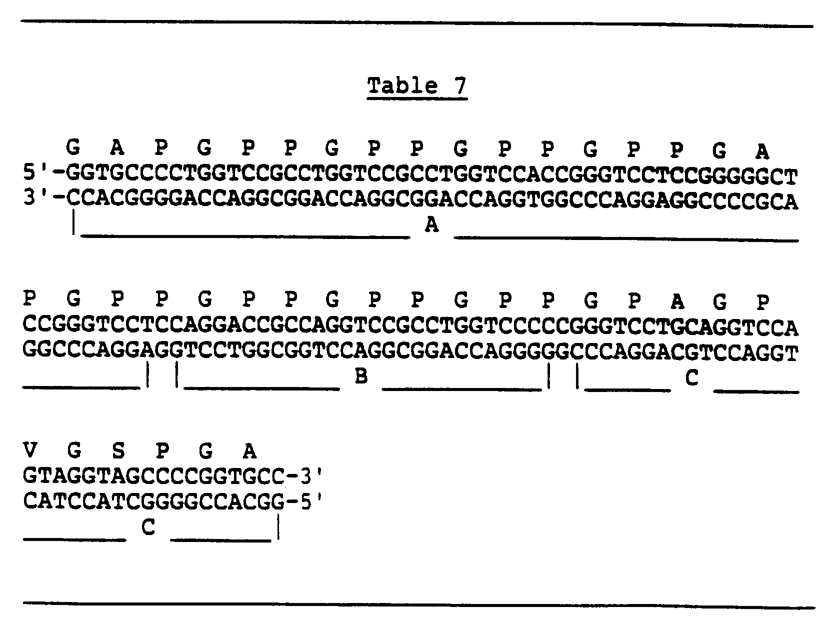

### Construction of CLP-CB monomer.

Two oligonucleotide strands were synthesized as described in Methods section.

AvaI                    PvuI
i)    GACTGCCAGGCCCGAAAGGCGATCGTGGCGACGCCGGTCCTAAAGGC
ii) GGCCCTGACGGTCCGGGCTTTCCGCTAGCACCGCTGCGGCCAGGATTTCCG

AvaI
GCAGATGGCAGCCCGG
CGTCTACCGTCG

The two oligonucleotide strands were annealed and ligated with the DNA of plasmid pPT0116 (CLP monomer in pSY937) which has been digested with AvaI REN.

The products of this ligation reaction were transformed into E. coli strain HB101. Plasmid DNA from transformants was purified and digested with BanI; clones containing inserts of the correct size were digested with PstI and BglI RENs for the determination of orientation. Plasmid DNA from correct clones was sequenced. Plasmid pPT0117 (shown in Table 8) contained the desired CLP-CB monomer sequence, oligonucleotides (i) and (ii) are shown below in bold and underlined.

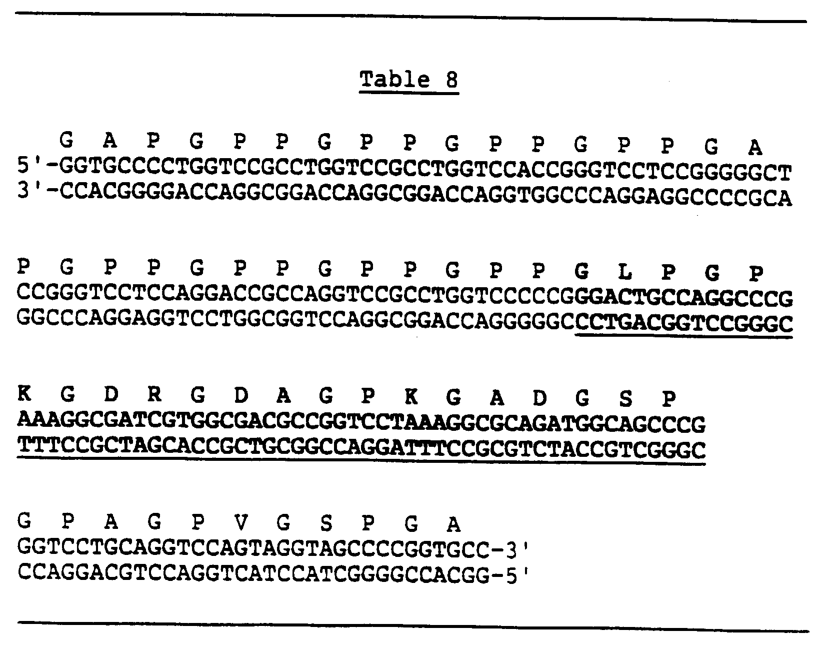

### CLP and CLP-CB Expression.

An overnight culture which had been grown at 30°C was used to inoculate 50 ml of media contained in a 250 ml flask. Kanamycin was added at a final concentration of 50 µg per ml and the culture was incubated with agitation (200 rpm) at 30°C. When the culture reached an OD₆₀₀ of 0.8, 40 ml were transferred to a new flask prewarmed at 42°C and incubated at the same temperature for approximately 2 hours. The cultures (30°C and 42°C) were chilled on ice and OD₆₀₀ was taken. Cells were collected by centrifugation divided in 1.0 OD₆₀₀ aliquot and used to perform dot blot and western analysis using CLP antisera (see Methods section). For purification and amino acid analysis larger cultures were used.

pPTO122 CLP Protein 760 AA MW 63,800
MDPVVLQRRDWENPGVTQLNRLAAHPPFASDPM
{[GAP(GPP)3]2GPVGSP}
GAMCAHRYQLSAGRYHYQLVWCQKZ

pPT0127    CLP-CB Protein    814 AA    MW 70,560
MDPVVLQRRDWENPGVTQLNRLAAHPPFASDPM
{[GAP(GPP)3]2(GLPGPKGDRGDAGPKGADGSPGPA)GPVGSP}
GAMCAHRYQLSAGRYHYQLVWCQKZ

Following the procedures described above, other polymers are prepared. These polymers influence cellular function by containing ligand sequences which interact with cell surface receptors. These polymers are prepared by inserting oligonucleotides encoding the nerve cell attachment promoting sequence from human laminin B1, YIGSR, into the protein polymer sequence of ELP I at the SmaI site. Additionally, a polymer is prepared by inserting oligonucleotides encoding the T cell receptor binding sequences from human MHC class I HLA-A2, PEYDGETRKVKAHSQTHRVDTLRY (residues 57-84) and TRKWAAHVEQLRAYEGTVEWRY (residues 143-171), into the SLPIII polymer in alternating fashion at the gene location containing the PstI site.

These polymers exhibit activities of chemical reactivity and cross-linking with themselves or with other polymers or materials. These polymers are prepared by inserting oligonucleotides encoding the sequences DPGK, NPGC, or RPGE into the PstI site of the synthetic polymer gene, SLPIII. Other examples of chemically reactive polymers are prepared by inserting oligonucleotides encoding the sequences GAGD, GAGC, or GAGK into the BanII site of the synthetic polymer gene, SLPIV, or by inserting oligonucleotides encoding the sequences GEGVP, GCGVP, or GKGVP into the SmaI site of the synthetic polymer gene ELP I. The synthetic polymers SLPIV and ELP I are described in the aforesaid PCT application.

Additional compositions are prepared according to the above procedures. These compositions include:

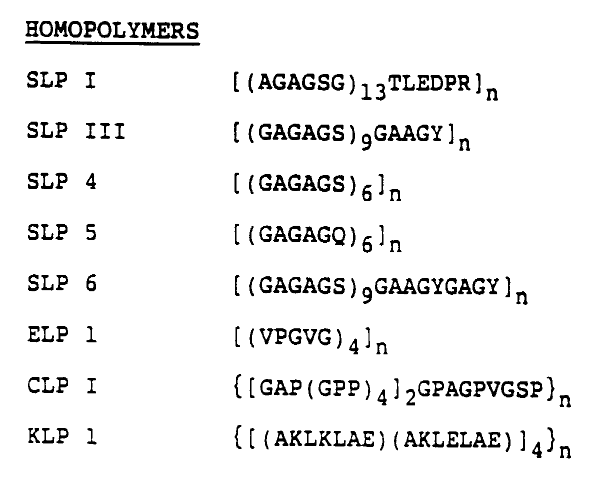

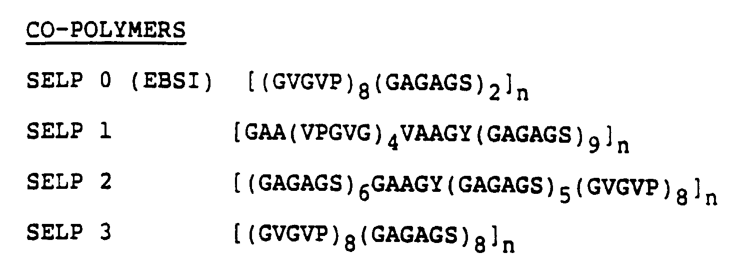

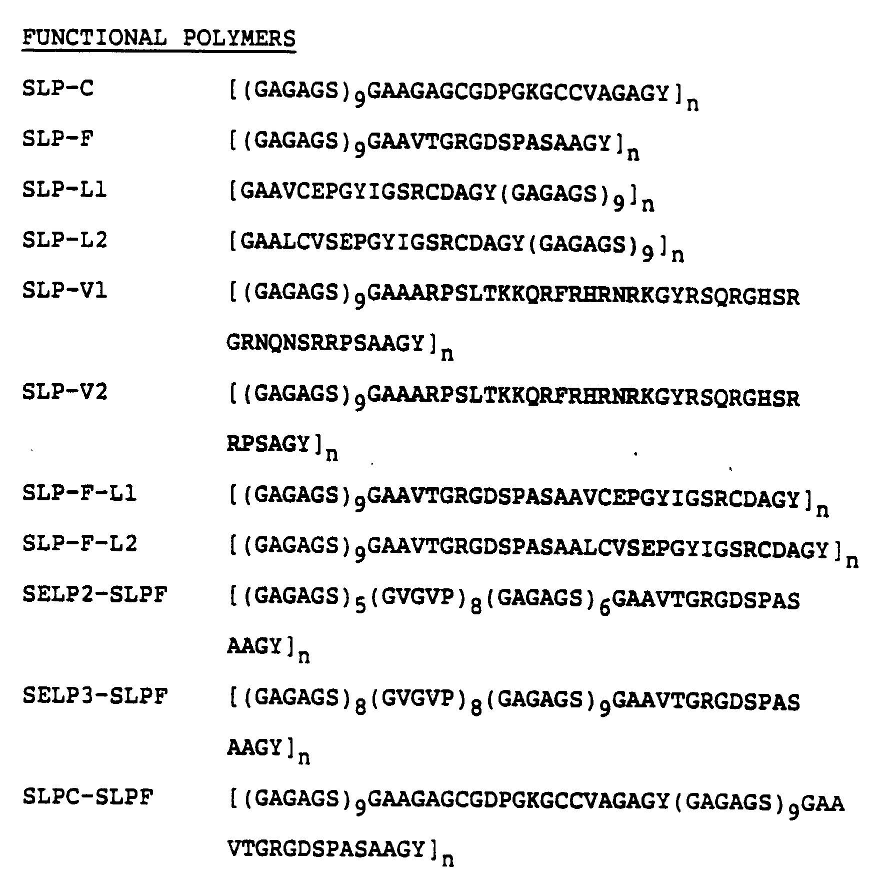

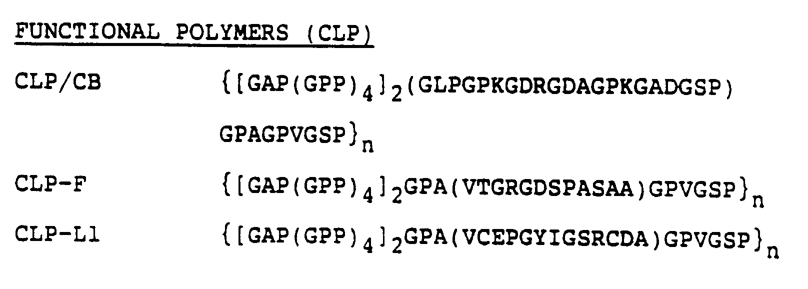

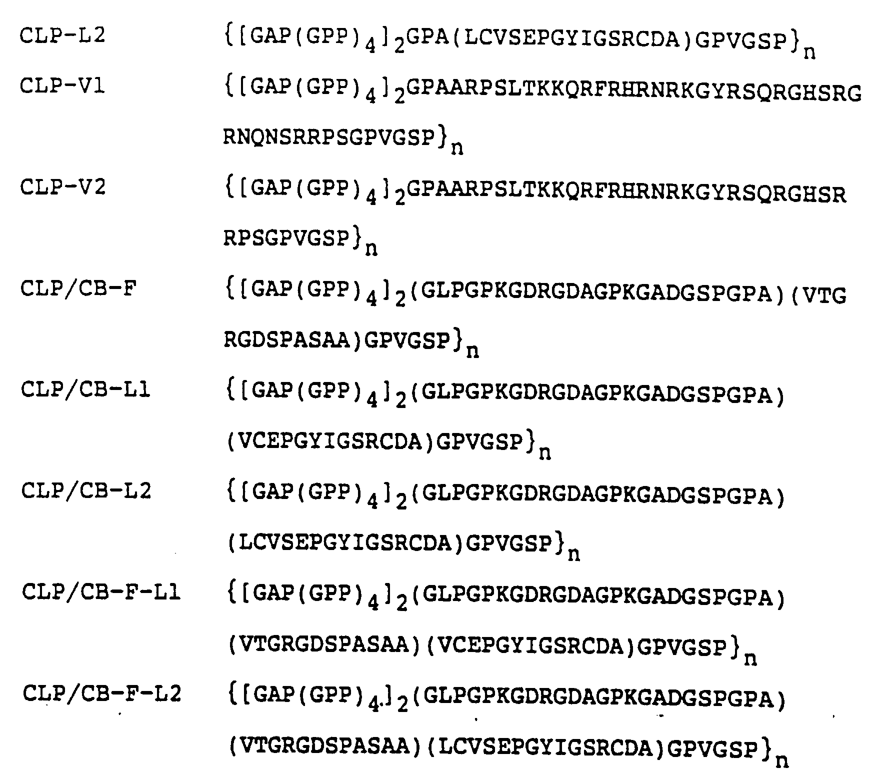

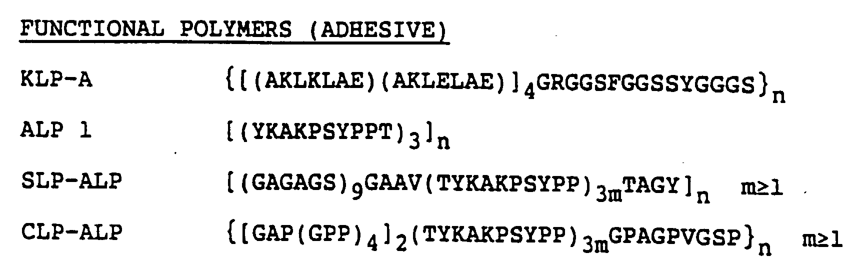

It is evident from the above results that a powerful capability is provided for producing polymers having good structural properties, as well as novel capabilities, where specific amino-acid sequences may be made readily available to the environment, while providing for structural capabilities such as fiber, film, and membrane formation. Thus the polymer is comprised of strands which interact to provide for structural characteristics, such as article formation with good tensile, e.g., mechanical properties, while at the same time providing for amino-acid sequences which may be used in a wide variety of biological and chemical applications. The subject compositions may be used for binding a wide variety of specific binding materials, as catalytic substances, where the amino-acid sequence may specifically chelate a wide variety of elements, as purification media, as composites, laminates, adhesives, combined with inorganic or organic materials, such as carbon fibers, nylon fibers, nitrocellulose, etc., as flask coatings for the growth of cells, as affinity columns, supports for biological materials, and the like.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto.

## Claims

1. A proteinaceous polymer comprising strands of repeating units capable of assembling into aligned structures to be formable into articles, with at least two strands joined by an intervening oligopeptide other than said repeating units, wherein said intervening oligopeptide is characterized by being unaligned and comprising a binding or chemically active amino acid sequence.

2. A polymer according to Claim 1, wherein said repeating units are repeating units of a natural polymer and from about 3 to 30 amino acids, said strands are of from about 25 to 150 amino acids, and said intervening oligopeptide is between each of said strands and is of from about 6 to 30 amino acids.

3. A polymer according to Claim 1, wherein said repeating unit is a repeating unit of fibroin, elastin, keratin, or collagen or combinations of multimers of said repeating units.

4. A polymer according to Claim 4, wherein said intervening oligopeptide includes the amino acid sequence RGD, DP, EP, DPGKGXY (wherein at least one of X and Y is C), EPGYIGSRCDAGY, PKGDRGDAGPK, or AVTGRDSPAS, where conservative substitutions may be made at other than the functional site.

5. A polymer according to Claim 1, wherein said polymer is a block copolymer comprising multimers of two different repeating units.

6. A proteinaceous polymer according to any one of the preceding claims wherein the repeating units have the sequence GAGAGS, VPGVG, or at least one of GPP and GAP.

7. A DNA sequence encoding a proteinaceous polymer comprising strands of repeating units capable of assembling into aligned structures to be formable into articles, with at least two strands joined by an intervening oligopeptide other than said repeating units, wherein said intervening oligopeptide is characterized by being unaligned and comprising a binding or chemically active amino acid sequence.

8. A DNA sequence according to Claim 7, wherein said repeating units are of a natural polymer and of from about 3 to 30 amino acids, said strands are of from about 25 to 150 amino acids, and said intervening oligopeptide is between each of said strands and is of from about 4 to 50 amino acids.

9. A DNA sequence according to Claim 7, wherein said repeating units have the sequence GAGAGS, VPGVG, or at least one of GPP and GAP or combinations of multimers of said repeating units.

10. A DNA sequence according to Claim 7, wherein said intervening oligopeptide includes the amino acid sequence RGD, DP, EP, DPGKGXY, wherein at least one of X and Y is C, EPGYIGSRCDAGY, PKGDRGDAGPK, or AVTGRDSPAS, where conservative substitutions may be made at other than the functional site.

11. A vector comprising a replication system and a DNA sequence according to Claim 7.

12. A vector according to Claim 11, wherein said replication system is a prokaryotic system.

13. A prokaryotic cell comprising a vector comprising a replication system and a DNA sequence according to Claim 7.

14. A method for producing a proteinaceous polymer comprising strands of repeating units capable of assembling into aligned structures to be formable into articles, with at least two strands joined by an intervening oligopeptide other than said repeating units, wherein said intervening oligopeptide is characterized by being unaligned, and comprising a binding or chemically active amino acid sequence, said method comprising:
growing in an appropriate nutrient medium a prokaryotic cell comprising a vector comprising a replication system functional in said prokaryotic cell and a gene capable of expression in said prokaryotic cell and encoding said proteinaceous polymer, whereby said proteinaceous polymer is expressed.

## Patentansprüche

1. Polymer vom Proteintyp, umfassend Stränge wiederkehrender Einheiten, die in der Lage sind, sich zu miteinander ausgerichteten Strukturen zusammenzusetzen, um zu Gegenständen formbar zu sein, wobei zumindest zwei Stränge durch ein dazwischenliegendes Oligopeptid miteinander verbunden sind, das anders ist als die wiederkehrenden Einheiten, worin das dazwischenliegende Oligopeptid dadurch gekennzeichnet ist, daß es nicht ausgerichtet ist und eine bindende oder chemisch aktive Aminosäuresequenz umfaßt.

2. Polymer nach Anspruch 1, worin die wiederkehrenden Einheiten wiederkehrende Einheiten eines natürlichen Polymers sind und etwa 3 bis 30 Aminosäuren umfassen, die Stränge etwa 25 bis 150 Aminosäuren umfassen und das dazwischenliegende Oligopeptid zwischen jedem der Stränge positioniert ist und etwa 6 bis 30 Aminosäuren umfaßt.

3. Polymer nach Anspruch 1, worin die wiederkehrende Einheit aus Fibroin, Elastin, Keratin oder Kollagen oder Kombinationen von Multimeren der wiederkehrenden Einheiten besteht.

4. Polymer nach Anspruch 4, worin das dazwischenliegende Oligopeptid die Aminosäuresequenz RGD, DP, EP, DPGKGXY (worin zumindest eines von X und Y = C ist), EPGYIGSRCDAGY, PKGDRGDAGPK oder AVTGRDSPAS umfaßt, worin konservative Substitutionen an einer anderen Position als an der funktionellen Stelle vorgenommen sein können.

5. Polymer nach Anspruch 1, worin das Polymer ein Blockcopolymer ist, das Multimere zweier unterschiedlicher wiederkehrender Einheiten umfaßt.

6. Proteinhältiges Polymer nach einem der vorhergehenden Ansprüche, worin die wiederkehrenden Einheiten die Sequenz GAGAGS, VPGVG oder zumindest eine von GPP und GAP aufweisen.

7. DNA-Sequenz, die für ein Polymer vom Proteintyp kodiert, umfassend Stränge wiederkehrender Einheiten, die in der Lage sind, sich zu ausgerichteten Strukturen zusammenzusetzen, die zu Gegenständen formbar sind, wobei zumindest zwei Stränge durch ein dazwischenliegendes Oligopeptid miteinander verbunden sind, das anders ist als die wiederkehrenden Einheiten, worin das dazwischenliegende Oligopeptid dadurch gekennzeichnet ist, daß es nicht ausgerichtet ist und eine bindende oder chemisch aktive Aminosäuresequenz umfaßt.

8. DNA-Sequenz nach Anspruch 7, worin die wiederkehrenden Einheiten wiederkehrende Einheiten eines natürlichen Polymers sind und etwa 3 bis 30 Aminosäuren umfassen, die Stränge etwa 25 bis 150 Aminosäuren umfassen und das dazwischenliegende Oligopeptid zwischen jedem Strang positioniert ist und etwa 4 bis 50 Aminosäuren umfaßt.

9. DNA-Sequenz nach Anspruch 7, worin die wiederkehrenden Einheiten die Sequenz GAGAGS, VPGVG oder zumindest eine von GPP und GAP oder Kombinationen von Multimeren der wiederkehrenden Einheiten aufweisen.

10. DNA-Sequenz nach Anspruch 7, worin das dazwischenliegende Oligopeptid die Aminosäuresequenz RGD, DP, EP, DPGKGXY, worin zumindest eines von X und Y = C ist, EPGYIGSRCDAGY, PKGDRGDAGPK oder AVTGRDSPAS umfaßt, worin konservative Substitutionen an einer anderen Position als der funktionellen Stelle vorgenommen sein können.

11. Vektor, umfassend ein Replikationssystem und eine DNA-Sequenz nach Anspruch 7.

12. Vektor nach Anspruch 11, worin das Replikationssystem ein prokaryotisches System ist.

13. Prokaryotische Zelle, umfassend einen Vektor, der ein Replikationssystem und eine DNA-Sequenz nach Anspruch 7 umfaßt.

14. Verfahren zur Herstellung eines Polymers vom Proteintyp, umfassend Stränge wiederkehrender Einheiten, die in der Lage sind, sich zu ausgerichteten Strukturen zusammenzusetzen, die zu Gegenständen formbar sind, wobei zumindest zwei Stränge durch ein dazwischenliegendes Oligopeptid miteinander verbunden sind, das anders ist als die wiederkehrenden Einheiten, worin das dazwischenliegende Oligopeptid dadurch gekennzeichnet ist, daß es nicht ausgerichtet ist und eine bindende oder chemisch aktive Aminosäuresequenz umfaßt, wobei das Verfahren folgendes umfaßt:
Züchten einer prokaryotischen Zelle in einem geeigneten Nährstoffmedium, wobei die Zelle einen Vektor umfaßt, der ein Replikationssystem, das in der prokaryotischen Zelle funktionell ist, und ein Gen umfaßt, das zur Expression in der prokaryotischen Zelle fähig ist und für das Polymer vom Proteintyp kodiert, wodurch das Polymer vom Proteintyp exprimiert wird.

## Revendications

1. Polymère protéique comprenant des brins d'unités récurrentes capables de s'assembler en des structures alignées pour pouvoir être formées en des articles, avec au moins deux brins joints par un oligopeptide intervenant différent desdites unités récurrentes, où ledit oligopeptide intervenant est caractérisé en ce qu'il est non-aligné et comprend une séquence d'acides aminés chimiquement active ou de liaison.

2. Polymère selon la revendication 1, dans lequel lesdites unités récurrentes sont des unités récurrentes d'un polymère naturel et d'environ 3 à 30 acides aminés, lesdits brins ont environ 25 à 150 acides aminés, et ledit oligopeptide intervenant est entre chacun desdits brins et a environ 6 à 30 acides aminés.

3. Polymère selon la revendication 1, dans lequel ladite unité récurrente est une unité récurrente de fibroïne, d'élastine, de kératine, de collagène ou des combinaisons de multimères desdites unités récurrentes.

4. Polymère selon la revendication 4, où ledit oligopeptide intervenant inclut la séquence d'acides aminés RGD, DP, EP, DPGKGXY (où au moins l'un de X et Y est C), EPGYIGSRCDAGY, PKGDRGDAGPK, ou AVTGRDSPAS, où des substitutions conservatives peuvent être effectuées à un site autre que le site fonctionnel.

5. Polymère selon la revendication 1, où ledit polymère est un copolymère bloc comprenant des multimères de deux unités récurrentes différentes.

6. Polymère protéique selon l'une quelconque des revendications précédentes, dans lequel les unités récurrentes ont la séquence GAGAGS, VPGVG, ou au moins l'une de GPP et GAP.

7. Séquence d'ADN encodant un polymère protéique comprenant des brins d'unités récurrentes capables de s'assembler en des structures alignées pour pouvoir être être formées en articles, avec au moins deux brins joints par un oligopeptide intervenant différent desdites unités récurrentes, dans laquelle ledit oligopeptide intervenant est caractérisé en ce qu'il est non-aligné et comprend une séquence d'acides aminés chimiquement active ou de liaison.

8. Séquence d'ADN selon la revendication 7, où lesdites unités récurrentes sont d'un polymère naturel et ont environ 3 à 30 acides aminés, lesdits brins ont environ 25 à 150 acides aminés, et ledit oligopeptide intervenant est entre chacun desdits brins et a environ 4 à 50 acides aminés.

9. Séquence d'ADN selon la revendication 7, dans laquelle lesdites unités récurrentes ont la séquence GAGAGS, VPGVG, ou au moins l'une de GPP et GAP ou des combinaisons de multimères desdites unités récurrentes.

10. Séquence d'ADN selon la revendication 7, dans laquelle ledit oligopeptide intervenant inclut la séquence d'acides aminés RGD, DP, EP, DPGKGXY, où au moins l'un de X et Y est C, EPGYIGSRCDAGY, PKGDRGDAGPK ou AVTGRDSPAS, où des substitutions conservatives ont été faites à un site autre que le site fonctionnel.

11. Vecteur comprenant un système de réplication et une séquence d'ADN selon la revendication 7.

12. Vecteur selon la revendication 11, où ledit système de réplication est un système procaryotique.

13. Cellule procaryotique comprenant un vecteur comprenant un système de réplication et une séquence d'ADN selon la revendication 7.

14. Méthode de production d'un polymère protéique comprenant des brins d'unités récurrentes capables de s'assembler en des structures alignées pour pouvoir être formées en des articles, avec au moins deux brins joints par un oligopeptide intervenant différent desdites unités récurrentes, où ledit oligopeptide intervenant est caractérisé en ce qu'il est non-aligné, et comprend une séquence d'acides aminés chimiquement active ou de liaison, ladite méthode comprenant :
la croissance dans un milieu de nutriment approprié d'un cellule procaryotique comprenant un vecteur comprenant un système de réplication fonctionnel dans ladite cellule procaryotique et un gène capable d'expression dans ladite cellule procaryotique et encodant ledit polymère protéique, ce par quoi ledit polymère protéique est exprimé.
